(19) 

**Europäisches Patentamt**

**European Patent Office**

**Office européen des brevets**

(11) **EP 4 397 686 A1**

(12) **EUROPEAN PATENT APPLICATION**
published in accordance with Art. 153(4) EPC

(43) Date of publication:
**10.07.2024 Bulletin 2024/28**

(21) Application number: **22863407.7**

(22) Date of filing: **29.08.2022**

(51) International Patent Classification (IPC):
*C07K 16/28* (2006.01)    *C07K 16/30* (2006.01)
*A61P 35/00* (2006.01)    *A61P 39/00* (2006.01)

(52) Cooperative Patent Classification (CPC):
**A61P 35/00; A61P 39/00; C07K 16/28; C07K 16/30**

(86) International application number:
**PCT/CN2022/115590**

(87) International publication number:
**WO 2023/030272 (09.03.2023 Gazette 2023/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.08.2021   CN 202111004998**

(71) Applicant: Oricell Therapeutics Co., Ltd.
**Shanghai 201203 (CN)**

(72) Inventors:
• **HE, Xiaowen**
**Shanghai 201203 (CN)**
• **ZHOU, Jincai**
**Shanghai 201203 (CN)**
• **CHEN, Siye**
**Shanghai 201203 (CN)**
• **YANG, Yue**
**Shanghai 201203 (CN)**
• **SHI, Zhongjun**
**Shanghai 201203 (CN)**
• **LI, Xiaopei**
**Shanghai 201203 (CN)**

(74) Representative: **Weickmann & Weickmann
PartmbB
Postfach 860 820
81635 München (DE)**

Remarks:
The complete document including Reference
Table(s) and the Sequence Listing(s) can be
downloaded from the EPO website

(54) **ANTI-GPRC5D ANTIGEN BINDING PROTEIN AND USE THEREOF**

(57)    The present application relates to an isolated antigen binding protein capable of binding to a G protein-coupled receptor, class C, group 5, member D (GPRC5D) protein with an $EC_{50}$ value of 1.0 $\mu$g/mL. The present application further relates to a chimeric antigen receptor comprising the antigen binding protein, and their uses in treatment of tumors.

EP 4 397 686 A1

**Description**

**FIELD OF THE INVENTION**

The present application relates to the field of biomedicine, and in particular to an anti-GPRC5D antigen binding protein, a chimeric antigen receptor comprising the antigen binding protein, and their uses in treatment of tumors.

**BACKGROUND OF THE INVENTION**

Multiple myeloma (MM) is the second most common malignant hematological disease and ranks second in terms of cancer mortality. MM is a plasmocyte malignant tumor that is often associated with multiple osteolytic lesions, renal impairment, bone marrow infiltrates, hypercalcemia, and anemia. At present, the main therapy for MM is systemic chemotherapy, which, however, has severe side effects and shows no prospect of permanent cure.

G protein-coupled receptor, class C, group 5, member D (GPRC5D) protein is an atypical surface orphan receptor. Like other C5 family receptors, GPRCD5 has a short amino terminal, and thus is conformationally very similar to the C4 family. GPRCD5's expression in normal tissues is only limited to hair follicles, but it is also highly expressed in the bone marrow of MM patients and is highly correlated with plasmocyte tumor burden and genetic aberrations.

With the introduction of new drugs such as protease inhibitors (PIs), immunomodulatory drugs (IMiD) and monoclonal antibodies (mAbs), the treatment of MM has been expanded, but still with very high recurrence rate and drug resistance. Chimeric antigen receptor T cell (CAR-T) therapy shows significant efficacy and controllable toxicity in MM, and the mainstream CAR-T cell therapy targeting B cell maturation antigens (BCMAs) has the problem of targeted escape for MM patients with negative BCMA or low BCMA expression. The high specific expression of GPRC5D on plasmocytes makes it an ideal therapeutic target for MM, but therapeutic drug products targeting GPRC5D haven't been well developed.

**SUMMARY OF THE INVENTION**

The present application provides an isolated antigen binding protein capable of binding to a G protein-coupled receptor, class C, group 5, member D (GPRC5D) protein. The present application further provides a chimeric antigen receptor comprising the antigen binding protein, and a cell comprising and/or expressing the chimeric antigen receptor. The cell has one or more of the following characteristics: (1) strong amplification ability, (2) ability to kill target cells expressing GPRC5D, (3) secretion of cytokines under target cell stimulation, (4) strong proliferative ability under target cell stimulation, and (4) inhibition of tumor growth.

In one aspect, the present application provides an isolated antigen binding protein capable of binding to a G protein-coupled receptor, class C, group 5, member D (GPRC5D) protein with an EC50 value below 1.0 $\mu$g/mL.

In some embodiments, the antigen binding protein comprises an antibody or an antigen binding fragment thereof.

In some embodiments, the antigen binding fragment comprises Fab, Fab', F(ab)2, a Fv fragment, F(ab')$_2$, scFv, di-scFv, VHH and/or dAb.

In some embodiments, the antigen binding fragment is VHH.

In some embodiments, the antibody is selected from the group consisting of: a monoclonal antibody, a chimeric antibody, a humanized antibody, and a fully human antibody.

In some embodiments, the antigen binding protein is capable of competing with a reference antibody for binding to the GPRC5D protein, wherein the reference antibody comprises an antibody heavy-chain variable region VH comprising HCDR1, HCDR2, and HCDR3, and the reference antibody comprises any set of amino acid sequences selected from the group consisting of:

(1) HCDR1: SEQ ID NO: 102, HCDR2: SEQ ID NO: 103, and HCDR3: SEQ ID NO: 104;

(2) HCDR1: SEQ ID NO: 105, HCDR2: SEQ ID NO: 106, and HCDR3: SEQ ID NO: 107;

(3) HCDR1: SEQ ID NO: 109, HCDR2: SEQ ID NO: 110, and HCDR3: SEQ ID NO: 111;

(4) HCDR1: SEQ ID NO: 105, HCDR2: SEQ ID NO: 114, and HCDR3: SEQ ID NO: 115;

(5) HCDR1: SEQ ID NO: 116, HCDR2: SEQ ID NO: 117, and HCDR3: SEQ ID NO: 118;

(6) HCDR1: SEQ ID NO: 119, HCDR2: SEQ ID NO: 120, and HCDR3: SEQ ID NO: 121;

(7) HCDR1: SEQ ID NO: 123, HCDR2: SEQ ID NO: 124, and HCDR3: SEQ ID NO: 125;

(8) HCDR1: SEQ ID NO: 127, HCDR2: SEQ ID NO: 128, and HCDR3: SEQ ID NO: 129;

(9) HCDR1: SEQ ID NO: 130, HCDR2: SEQ ID NO: 131, and HCDR3: SEQ ID NO: 132;

(10) HCDR1: SEQ ID NO: 95, HCDR2: SEQ ID NO: 96, and HCDR3: SEQ ID NO: 97;

(11) HCDR1: SEQ ID NO: 133, HCDR2: SEQ ID NO: 134, and HCDR3: SEQ ID NO: 135;

(12) HCDR1: SEQ ID NO: 136, HCDR2: SEQ ID NO: 134, and HCDR3: SEQ ID NO: 137;

(13) HCDR1: SEQ ID NO: 138, HCDR2: SEQ ID NO: 139, and HCDR3: SEQ ID NO: 140;

(14) HCDR1: SEQ ID NO: 142, HCDR2: SEQ ID NO: 143, and HCDR3: SEQ ID NO: 144;

(15) HCDR1: SEQ ID NO: 145, HCDR2: SEQ ID NO: 146, and HCDR3: SEQ ID NO: 147;

(16) HCDR1: SEQ ID NO: 148, HCDR2: SEQ ID NO: 149, and HCDR3: SEQ ID NO: 150;

(17) HCDR1: SEQ ID NO: 152, HCDR2: SEQ ID NO: 153, and HCDR3: SEQ ID NO: 154;

(18) HCDR1: SEQ ID NO: 123, HCDR2: SEQ ID NO: 155, and HCDR3: SEQ ID NO: 156;

(19) HCDR1: SEQ ID NO: 157, HCDR2: SEQ ID NO: 153, and HCDR3: SEQ ID NO: 158;

(20) HCDR1: SEQ ID NO: 159, HCDR2: SEQ ID NO: 160, and HCDR3: SEQ ID NO: 161;

(21) HCDR1: SEQ ID NO: 159, HCDR2: SEQ ID NO: 162, and HCDR3: SEQ ID NO: 163;

(22) HCDR1: SEQ ID NO: 159, HCDR2: SEQ ID NO: 164, and HCDR3: SEQ ID NO: 165;

(23) HCDR1: SEQ ID NO: 166, HCDR2: SEQ ID NO: 167, and HCDR3: SEQ ID NO: 168;

(24) HCDR1: SEQ ID NO: 169, HCDR2: SEQ ID NO: 153, and HCDR3: SEQ ID NO: 170;

(25) HCDR1: SEQ ID NO: 157, HCDR2: SEQ ID NO: 171, and HCDR3: SEQ ID NO: 172;

(26) HCDR1: SEQ ID NO: 174, HCDR2: SEQ ID NO: 175, and HCDR3: SEQ ID NO: 176;

(27) HCDR1: SEQ ID NO: 177, HCDR2: SEQ ID NO: 178, and HCDR3: SEQ ID NO: 179;

(28) HCDR1: SEQ ID NO: 180, HCDR2: SEQ ID NO: 181, and HCDR3: SEQ ID NO: 182;

(29) HCDR1: SEQ ID NO: 184, HCDR2: SEQ ID NO: 106, and HCDR3: SEQ ID NO: 185;

(30) HCDR1: SEQ ID NO: 186, HCDR2: SEQ ID NO: 187, and HCDR3: SEQ ID NO: 188;

(31) HCDR1: SEQ ID NO: 189, HCDR2: SEQ ID NO: 190, and HCDR3: SEQ ID NO: 191;

(32) HCDR1: SEQ ID NO: 159, HCDR2: SEQ ID NO: 192, and HCDR3: SEQ ID NO: 179;

(33) HCDR1: SEQ ID NO: 193, HCDR2: SEQ ID NO: 194, and HCDR3: SEQ ID NO: 195; and

(34) HCDR1: SEQ ID NO: 196, HCDR2: SEQ ID NO: 197, and HCDR3: SEQ ID NO: 198.

In some embodiments, the antigen binding protein comprises at least one CDR derived from the antibody heavy-chain variable region VH, the VH comprising an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

In some embodiments, the antigen binding protein comprises HCDR3, and the HCDR3 comprises an amino acid sequence as set forth in any one of SEQ ID NO: 215 or SEQ ID NO: 218.

In some embodiments, the antigen binding protein comprises HCDR3, and the HCDR3 comprises an amino acid sequence as set forth in any one of SEQ ID NO: 97, SEQ ID NO: 104, SEQ ID NO: 107, SEQ ID NO: 111, SEQ ID NO: 115, SEQ ID NO: 118, SEQ ID NO: 121, SEQ ID NO: 125, SEQ ID NO: 129, SEQ ID NO: 132, SEQ ID NO: 135, SEQ ID NO: 137, SEQ ID NO: 140, SEQ ID NO: 144, SEQ ID NO: 147, SEQ ID NO: 150, SEQ ID NO: 154, SEQ ID NO: 156, SEQ ID NO: 158, SEQ ID NO: 161, SEQ ID NO: 163, SEQ ID NO: 165, SEQ ID NO: 168, SEQ ID NO: 170, SEQ ID NO: 172, SEQ ID NO: 176, SEQ ID NO: 179, SEQ ID NO: 182, SEQ ID NO: 185, SEQ ID NO: 188, SEQ ID NO: 191, SEQ ID NO: 195 and SEQ ID NO: 198.

In some embodiments, the antigen binding protein comprises HCDR2, and the HCDR2 comprises an amino acid sequence as set forth in $X_1X_2X_3X_4X_5X_6X_7T$, wherein $X_1$ is I or T, $X_2$ is N, S or T, $X_3$ is A, P, R, S or W, $X_4$ is G, R, S, T or none, $X_5$ is D or G, $X_6$ is G or S, and $X_7$ is D, G, I, N, R, S, T or V

In some embodiments, the antigen binding protein comprises HCDR2, and the HCDR2 comprises an amino acid sequence as set forth in any one of SEQ ID NO: 96, SEQ ID NO: 103, SEQ ID NO: 106, SEQ ID NO: 110, SEQ ID NO: 114, SEQ ID NO: 117, SEQ ID NO: 120, SEQ ID NO: 124, SEQ ID NO: 128, SEQ ID NO: 131, SEQ ID NO: 134, SEQ ID NO: 139, SEQ ID NO: 143, SEQ ID NO: 146, SEQ ID NO: 149, SEQ ID NO: 153, SEQ ID NO: 155, SEQ ID NO: 160, SEQ ID NO: 162, SEQ ID NO: 164, SEQ ID NO: 167, SEQ ID NO: 171, SEQ ID NO: 175, SEQ ID NO: 178, SEQ ID NO: 181, SEQ ID NO: 187, SEQ ID NO: 190, SEQ ID NO: 192, SEQ ID NO: 194 and SEQ ID NO: 197.

In some embodiments, the antigen binding protein comprises HCDR1, and the HCDR1 comprises an amino acid sequence as set forth in $GX_2X_3X_4SX_6X_7X_8$, wherein $X_2$ is F, G, I, L, N, R, S or Y, $X_3$ is I or T, $X_4$ is F or L, $X_6$ is I, L, N, R, S, TorY, $X_7$ is D, N or Y, and $X_8$ is A, D, G, I, N, R, S, T, V or Y

In some embodiments, the antigen binding protein comprises HCDR1, and the HCDR1 comprises an amino acid sequence as set forth in any one of SEQ ID NO: 95, SEQ ID NO: 102, SEQ ID NO: 105, SEQ ID NO: 109, SEQ ID NO: 116, SEQ ID NO: 119, SEQ ID NO: 123, SEQ ID NO: 127, SEQ ID NO: 130, SEQ ID NO: 133, SEQ ID NO: 136, SEQ ID NO: 138, SEQ ID NO: 142, SEQ ID NO: 145, SEQ ID NO: 148, SEQ ID NO: 152, SEQ ID NO: 157, SEQ ID NO: 159, SEQ ID NO: 166, SEQ ID NO: 169, SEQ ID NO: 174, SEQ ID NO: 177, SEQ ID NO: 180, SEQ ID NO: 184, SEQ ID NO: 186, SEQ ID NO: 189, SEQ ID NO: 193 and SEQ ID NO: 196.

In some embodiments, the antigen binding protein comprises HCDR1, HCDR2, and HCDR3, and the HCDR1, HCDR2, and HCDR3 comprise any set of amino acid sequences selected from the group consisting of:

(1) HCDR1: SEQ ID NO: 102, HCDR2: SEQ ID NO: 103, and HCDR3: SEQ ID NO: 104;
(2) HCDR1: SEQ ID NO: 105, HCDR2: SEQ ID NO: 106, and HCDR3: SEQ ID NO: 107;
(3) HCDR1: SEQ ID NO: 109, HCDR2: SEQ ID NO: 110, and HCDR3: SEQ ID NO: 111;
(4) HCDR1: SEQ ID NO: 105, HCDR2: SEQ ID NO: 114, and HCDR3: SEQ ID NO: 115;
(5) HCDR1: SEQ ID NO: 116, HCDR2: SEQ ID NO: 117, and HCDR3: SEQ ID NO: 118;
(6) HCDR1: SEQ ID NO: 119, HCDR2: SEQ ID NO: 120, and HCDR3: SEQ ID NO: 121;
(7) HCDR1: SEQ ID NO: 123, HCDR2: SEQ ID NO: 124, and HCDR3: SEQ ID NO: 125;
(8) HCDR1: SEQ ID NO: 127, HCDR2: SEQ ID NO: 128, and HCDR3: SEQ ID NO: 129;
(9) HCDR1: SEQ ID NO: 130, HCDR2: SEQ ID NO: 131, and HCDR3: SEQ ID NO: 132;
(10) HCDR1: SEQ ID NO: 95, HCDR2: SEQ ID NO: 96, and HCDR3: SEQ ID NO: 97;
(11) HCDR1: SEQ ID NO: 133, HCDR2: SEQ ID NO: 134, and HCDR3: SEQ ID NO: 135;
(12) HCDR1: SEQ ID NO: 136, HCDR2: SEQ ID NO: 134, and HCDR3: SEQ ID NO: 137;
(13) HCDR1: SEQ ID NO: 138, HCDR2: SEQ ID NO: 139, and HCDR3: SEQ ID NO: 140;
(14) HCDR1: SEQ ID NO: 142, HCDR2: SEQ ID NO: 143, and HCDR3: SEQ ID NO: 144;
(15) HCDR1: SEQ ID NO: 145, HCDR2: SEQ ID NO: 146, and HCDR3: SEQ ID NO: 147;
(16) HCDR1: SEQ ID NO: 148, HCDR2: SEQ ID NO: 149, and HCDR3: SEQ ID NO: 150;
(17) HCDR1: SEQ ID NO: 152, HCDR2: SEQ ID NO: 153, and HCDR3: SEQ ID NO: 154;
(18) HCDR1: SEQ ID NO: 123, HCDR2: SEQ ID NO: 155, and HCDR3: SEQ ID NO: 156;
(19) HCDR1: SEQ ID NO: 157, HCDR2: SEQ ID NO: 153, and HCDR3: SEQ ID NO: 158;
(20) HCDR1: SEQ ID NO: 159, HCDR2: SEQ ID NO: 160, and HCDR3: SEQ ID NO: 161;
(21) HCDR1: SEQ ID NO: 159, HCDR2: SEQ ID NO: 162, and HCDR3: SEQ ID NO: 163;
(22) HCDR1: SEQ ID NO: 159, HCDR2: SEQ ID NO: 164, and HCDR3: SEQ ID NO: 165;
(23) HCDR1: SEQ ID NO: 166, HCDR2: SEQ ID NO: 167, and HCDR3: SEQ ID NO: 168;

(24) HCDR1: SEQ ID NO: 169, HCDR2: SEQ ID NO: 153, and HCDR3: SEQ ID NO: 170;
(25) HCDR1: SEQ ID NO: 157, HCDR2: SEQ ID NO: 171, and HCDR3: SEQ ID NO: 172;
(26) HCDR1: SEQ ID NO: 174, HCDR2: SEQ ID NO: 175, and HCDR3: SEQ ID NO: 176;
(27) HCDR1: SEQ ID NO: 177, HCDR2: SEQ ID NO: 178, and HCDR3: SEQ ID NO: 179;
(28) HCDR1: SEQ ID NO: 180, HCDR2: SEQ ID NO: 181, and HCDR3: SEQ ID NO: 182;
(29) HCDR1: SEQ ID NO: 184, HCDR2: SEQ ID NO: 106, and HCDR3: SEQ ID NO: 185;
(30) HCDR1: SEQ ID NO: 186, HCDR2: SEQ ID NO: 187, and HCDR3: SEQ ID NO: 188;
(31) HCDR1: SEQ ID NO: 189, HCDR2: SEQ ID NO: 190, and HCDR3: SEQ ID NO: 191;
(32) HCDR1: SEQ ID NO: 159, HCDR2: SEQ ID NO: 192, and HCDR3: SEQ ID NO: 179;
(33) HCDR1: SEQ ID NO: 193, HCDR2: SEQ ID NO: 194, and HCDR3: SEQ ID NO: 195; and
(34) HCDR1: SEQ ID NO: 196, HCDR2: SEQ ID NO: 197, and HCDR3: SEQ ID NO: 198.

In some embodiments, the antigen binding protein comprises an antibody heavy-chain variable region VH, wherein the VH comprises a framework region H-FR1, a C-terminal of the H-FR1 is directly or indirectly linked to an N-terminal of the HCDR1, and the H-FR1 comprises an amino acid sequence as set forth in SEQ ID NO: 98.
In some embodiments, the VH in the antigen binding protein comprises a framework region H-FR2 located between the HCDR1 and the HCDR2, and the H-FR2 comprises an amino acid sequence as set forth in SEQ ID NO: 216.
In some embodiments, the H-FR2 in the antigen binding protein comprises an amino acid sequence as set forth in SEQ ID NO: 99, SEQ ID NO: 108, SEQ ID NO: 112, SEQ ID NO: 122, SEQ ID NO: 151, and SEQ ID NO: 173.
In some embodiments, the VH in the antigen binding protein comprises a framework region H-FR3 located between the HCDR2 and the HCDR3, and the H-FR3 comprises an amino acid sequence as set forth in SEQ ID NO: 217.
In some embodiments, the H-FR3 in the antigen binding protein comprises an amino acid sequence as set forth in any one of SEQ ID NO: 100, SEQ ID NO: 113, SEQ ID NO: 126, SEQ ID NO: 141, and SEQ ID NO: 183.
In some embodiments, the VH in the antigen binding protein comprises a framework region H-FR4, an N-terminal of the H-FR4 is linked to a C-terminal of the HCDR3, and the H-FR4 comprises an amino acid sequence as set forth in SEQ ID NO: 101.
In some embodiments, the VH in the antigen binding protein comprises an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.
In some embodiments, the antigen binding protein is VHH, and the VHH comprises an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.
In some embodiments, the antigen binding protein comprises an antibody heavy-chain constant region derived from IgG.
In some embodiments, the antigen binding protein comprises an antibody heavy-chain constant region derived from human IgG.
In some embodiments, the antigen binding protein comprises an antibody heavy-chain constant region derived from human IgG1.
In another aspect, the present application provides a chimeric antigen receptor comprising a targeting moiety, the targeting moiety comprising the antigen binding protein.
In some embodiments, the chimeric antigen receptor comprises a co-stimulatory signal region, wherein the co-stimulatory signal region comprises an intracellular co-stimulatory signal region derived from one or more proteins selected from the group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD83 ligands, CD40, and MyD88.
In some embodiments, the co-stimulatory signal region in the chimeric antigen receptor is a 4-1BB-derived intracellular co-stimulatory signal region.
In some embodiments, the co-stimulatory signal region in the chimeric antigen receptor comprises an amino acid sequence as set forth in SEQ ID NO: 78.
In some embodiments, the chimeric antigen receptor comprises an intracellular signal region, the intracellular signal region comprising an intracellular signal region derived from one or more proteins selected from the group consisting of: CD3ζ, CD3δ, CD3γ, CD3ε, CD79a, CD79b, FcεRIγ, FcεRIβ, FcγRIIa, bovine leukemia virus gp30, Epstein-Barr virus

(EBV) LMP2A, simian immunodeficiency virus PBj 14 Nef, Kaposi sarcoma-associated herpesvirus (HSKV), DAP10, DAP-12 and a domain comprising at least one ITAM domain.

In some embodiments, the intracellular signal region in the chimeric antigen receptor is a CD3$\zeta$-derived signaling domain.

In some embodiments, the intracellular signal region in the chimeric antigen receptor comprises an amino acid sequence as set forth in SEQ ID NO: 80.

In some embodiments, the chimeric antigen receptor comprises a transmembrane region, the transmembrane region comprising a transmembrane region derived from one or more proteins selected from the group consisting of: CD8, CD28, 4-1BB, CD4, CD27, CD7, PD-1, TRAC, TRBC, CD3$\varepsilon$, CD3$\zeta$, CTLA-4, LAG-3, CD5, ICOS, OX40, NKG2D, 2B4, CD244, Fc$\varepsilon$RI$\gamma$, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154 and SLAM.

In some embodiments, the transmembrane region in the chimeric antigen receptor is a CD8-derived transmembrane region.

In some embodiments, the transmembrane region in the chimeric antigen receptor comprises an amino acid sequence as set forth in SEQ ID NO: 76.

In some embodiments, the chimeric antigen receptor comprises a hinge region between the targeting moiety and the transmembrane region, the hinge region comprising a hinge region derived from one or more proteins selected from the group consisting of: CD28, IgG1, IgG4, IgD, 4-1BB, CD4, CD27, CD7, CD8, PD-1, ICOS, OX40, NKG2D, NKG2C, Fc$\varepsilon$RI$\gamma$, BTLA, GITR, DAP10, CD40L, TIM1, CD226, SLAM, CD30 and LIGHT.

In some embodiments, the hinge region in the chimeric antigen receptor is a CD8-derived hinge region.

In some embodiments, the hinge region in the chimeric antigen receptor comprises an amino acid sequence as set forth in SEQ ID NO: 74.

In some embodiments, the chimeric antigen receptor further comprises a low-density lipoprotein receptor-related protein or a fragment thereof, the low-density lipoprotein receptor-related protein or the fragment thereof being located at the C-terminal of the intracellular signal region.

In some embodiments, the low-density lipoprotein receptor-related protein or the fragment thereof in the chimeric antigen receptor comprises one or more selected from the group consisting of: low-density lipoprotein receptor-related proteins 1-12 or fragments thereof.

In some embodiments, the low-density lipoprotein receptor-related protein or the fragment thereof in the chimeric antigen receptor is a low-density lipoprotein receptor-related protein 6 or a fragment thereof.

In some embodiments, the low-density lipoprotein receptor-related protein or the fragment thereof in the chimeric antigen receptor comprises an amino acid sequence as set forth in SEQ ID NO: 84.

In some embodiments, the chimeric antigen receptor further comprises a signal peptide.

In some embodiments, the signal peptide in the chimeric antigen receptor is a CD8 protein-derived signal peptide.

In some embodiments, the signal peptide in the chimeric antigen receptor comprises an amino acid sequence as set forth in SEQ ID NO: 72.

In some embodiments, the chimeric antigen receptor comprises an amino acid sequence as set forth in any one of SEQ ID NO: 86, SEQ ID NO: 88, SEQ ID NO: 90, SEQ ID NO: 92, SEQ ID NO: 200, SEQ ID NO: 201, SEQ ID NO: 202, SEQ ID NO: 203 and SEQ ID NO: 204.

In some embodiments, the chimeric antigen receptor comprises a nucleotide sequence as set forth in any one of SEQ ID NO: 85, SEQ ID NO: 87, SEQ ID NO: 89, and SEQ ID NO: 91.

In another aspect, the present application further provides a polypeptide, comprising the antigen binding protein.

In another aspect, the present application further provides an isolated nucleic acid molecule or isolated nucleic acid molecules, encoding the antigen binding protein and/or the chimeric antigen receptor.

In some embodiments, the nucleic acid molecule further comprises a promoter.

In some embodiments, the promoter in the nucleic acid molecule is a constitutive promoter.

In some embodiments, the promoter in the nucleic acid molecule is an EF1$\alpha$ promoter.

In some embodiments, the nucleic acid molecule comprises a nucleotide sequence as set forth in any one of SEQ ID NO: 85, SEQ ID NO: 87, SEQ ID NO: 89, and SEQ ID NO: 91.

In another aspect, the present application further provides a vector, comprising the nucleic acid molecule.

In some embodiments, the vector is a viral vector.

In some embodiments, the vector is a lentiviral vector.

In another aspect, the present application further provides a cell, comprising the antigen binding protein, the chimeric antigen receptor, the nucleic acid molecule and/or the vector.

In some embodiments, the cell is an immune effector cell.

In some embodiments, the cell comprises a T cell, a B cell, a natural killer (NK) cell, a macrophage, a NKT cell, a monocyte, a dendritic cell, a granulocyte, a lymphocyte, a leukocyte, a peripheral blood mononuclear cell, an embryonic stem cell, a lymphoprogenitor cell, and/or a pluripotent stem cell.

In some embodiments, the cell is a T cell.

In another aspect, the present application further provides a method for preparing the antigen binding protein and/or the chimeric antigen receptor, wherein the method comprises culturing the cell under a condition enabling expression of the antigen binding protein and/or the chimeric antigen receptor.

In another aspect, the present application further provides a method for preparing a modified immune effector cell, comprising introducing the vector into an immune effector cell.

In another aspect, the present application further provides a pharmaceutical composition, comprising the antigen binding protein, the chimeric antigen receptor, the polypeptide, the nucleic acid molecule, the vector, and/or the cell, and optionally a pharmaceutically acceptable carrier.

In another aspect, the present application further provides use of the antigen binding protein, the chimeric antigen receptor, the nucleic acid molecule, the vector, the cell, and/or the pharmaceutical composition in preparation of a medicament for treating, preventing and/or relieving diseases or disorders associated with abnormal expression of GPRC5D.

In some embodiments, the diseases or disorders associated with abnormal expression of GPRC5D in the use comprise tumors.

In some embodiments, the tumors in the use comprise solid tumors.

In some embodiments, the tumors in the use comprise non-solid tumors.

In some embodiments, the tumors in the use comprise hematologic neoplasms and/or lymphomas.

In some embodiments, the tumors in the use comprise myelomas.

In another aspect, the present application further provides a method for preventing, treating and/or relieving diseases or disorders associated with abnormal expression of GPRC5D, comprising administering the antigen binding protein, the chimeric antigen receptor, the nucleic acid molecule, the vector, the cell, and/or the pharmaceutical composition to a subject in need thereof.

In some embodiments, the diseases or disorders associated with abnormal expression of GPRC5D in the method comprise tumors.

In some embodiments, the tumors in the method comprise solid tumors.

In some embodiments, the tumors in the method comprise non-solid tumors.

In some embodiments, the tumors in the method comprise hematologic neoplasms and/or lymphomas.

In some embodiments, the tumors in the method comprise myelomas.

Other aspects and advantages of the present application may be readily perceived by those skilled in the art from the detailed description below. The detailed description below only illustrates and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

## BRIEF DESCRIPTION OF THE DRAWINGS

The specific features of the invention involved in the present application are listed in the appended claims. The characteristics and advantages of the invention involved in the present application may be better understood by referring to the exemplary embodiments and the accompanying drawings described in detail below. The accompanying drawings are briefly illustrated as follows.

FIGs. 1A to 1J show the binding activity of the anti-CPRC5D antibody of the present application to the cell surface GPRC5D under flow cytometry.

FIG. 2 shows the elements and ligation sequences of CAR components in the chimeric antigen receptor lentiviral expression vector and the GPRC5D core plasmid according to the present application.

FIGs. 3A to 3C show the amplification factor of activated cultured GPRC5D CAR-T cells and the CAR positive rate of infected T cells.

FIGs. 4A to 4D show the killing ability of GPRC5D CAR-T cells against target cells (CHO cells/MM1S cells).

FIGs. 5A to 5B show the secretion of cytokines (IL2\IFN-$\gamma$) after in vitro killing by GPRC5D CAR-T cells.

FIG. 6 shows the targeted proliferation rate of GPRC5D CAR-T cells.

FIGs. 7A to 7D show the cytokine secretion, tumor reduction ability and safety of GPRC5D CAR-T cells in mice.

FIGs. 8A to 8D and FIGs. 9A to 9D show that the anti-GPRC5D antibody does not bind to cells that do not express the GPRC5D antigen, with A: A549 cells, B: KERA cells, C: MCF-7 cells, and D: MSC cells.

FIGs. 10A to 10D show the assay results of anti-GPRC5D endocytosis.

## DETAILED DESCRIPTION OF THE PREFERRED EMBODIMENTS

The embodiments of the invention of the present application will be explained by specific examples below. Those familiar with this technology may readily understand other advantages and effects of the invention of the present application from the content disclosed in the specification.

## TERMS & DEFINITIONS

In the present application, the term "isolated antigen binding protein" generally refers to a protein that has been removed from its naturally occurring state and is capable of binding to an antigen. The "isolated antigen binding protein" may comprise an antigen binding moiety and optionally, a framework or construct moiety allowing the antigen binding moiety to use a conformation that facilitates its binding to an antigen. The antigen binding protein may comprise, for example, an antibody-derived protein framework region (FR) or alternative protein or artificial framework region having a grafted variable region (CDR) or a CDR derivative. In the present application, the antigen binding protein may comprise an antibody or an antigen binding fragment thereof. For example, the antigen binding protein may bind to GPRC5D protein. For example, the antigen binding protein may compete with a reference antibody for binding to a GPRC5D protein. For example, the antigen binding protein may comprise an antibody heavy-chain variable region VH. For example, the antigen binding protein may comprise at least one CDR derived from the antibody heavy-chain variable region VH. For example, the VH may comprise HCDR3, HCDR2 and/or HCDR1. In the present application, the VH may comprise a framework region H-FR1, and a C-terminal of the H-FR1 is directly or indirectly linked to an N-terminal of the HCDR1. For example, the VH may comprise a framework region H-FR2 located between the HCDR1 and the HCDR2. For example, the VH may comprise a framework region H-FR3 located between the HCDR2 and the HCDR3. For example, the VH may comprise a framework region H-FR4, and a C-terminal of the H-FR4 is directly or indirectly linked to an N-terminal of the HCDR3. For example, the antigen binding protein may be VHH. For example, the antigen binding protein may comprise an antibody heavy-chain constant region, which may be derived from IgG. For example, the antibody heavy-chain constant region may be derived from human IgG. For example, the antibody heavy-chain constant region may be derived from human IgG1.

The term "antibody" used comprises an intact antibody and a binding fragment thereof. Generally, the fragment competes with the intact antibody, from which it is derived, for binding specifically to an antigen. Optionally, the antibody or the binding fragment thereof may chemically bind to other proteins, or may be expressed as a fusion protein together with other proteins. For example, the antibody may be a monoclonal antibody, a chimeric antibody, a humanized antibody and a fully human antibody. For example, the binding protein of the antibody or the binding fragment thereof may comprise GPRC5D. For example, the antibody or the binding fragment thereof may be specific for GPRC5D.

The term "antigen binding fragment" refers to a portion of an intact antibody and refers to an antigen-determining variable region of the intact antibody. For example, the antigen binding fragment may comprise Fab, Fab', F(ab')2, a Fv fragment and a single-stranded Fv fragment, a tandem Fv fragment, VHH, and a bispecific antibody. For example, the antigen binding fragment may be VHH. For example, the antigen binding fragment may bind to VHH. For example, the antigen binding fragment may be specific for GPRC5D.

In the present application, the term "VHH" generally refers to an antibody that comprises a variable antigen-binding domain of a heavy-chain antibody. VHH may also be called nanobody (Nb) and/or single-domain antibody. For example, the VHH may bind to GPRC5D. For example, the VHH may be specific for GPRC5D.

In the present application, the antibody may comprise at least two heavy (H) chains and two light (L) chains, which are interlinked by disulfide bonds. Each heavy chain consists of a heavy-chain variable region (VH) and a heavy-chain constant region. The term "heavy-chain constant region" consists of three domains CH1, CH2 and CH3. Each light chain consists of a light-chain variable region (VL) and a light-chain constant region. The term "light-chain constant region" consists of one domain CL. The VH and VL may be further subdivided into highly variable regions known as complementary determining regions (CDRs), with the interspersal of more conservative regions known as frame work regions (FR). Each of VH and VL consists of three CDRs and four FRs arranged from an amino terminal to a carboxyl terminal in the following order: FR1, CDR1, FR2, CDR2, FR3, CDR3, and FR4. The heavy-chain and light-chain variable regions comprise binding domains that interact with antigens. The constant region of the antibody may mediate the binding of immunoglobulins to host tissues or factors.

In the present application, the term "reference antibody" refers to an antibody that can compete with the isolated antigen binding protein for binding to the same epitope. The reference antibody may comprise a heavy-chain variable region VH. For example, the reference antibody may have 3 CDR sequences. For example, the VH of the reference antibody may comprise HCDR1, HCDR2, and HCDR3. For example, the CDR sequences may be the same as the CDR sequences of the isolated antigen binding protein.

In the present application, the term "G protein-coupled receptor, class C, group 5, member D (GPRC5D) protein" is an atypical surface orphan receptor. Like other C5 family receptors, GPRCD5 has a short amino terminal, and thus is conformationally very similar to the C4 family. GPRCD5's expression in normal tissues is only limited to hair follicles, but it is also significantly expressed in the bone marrow of multiple myeloma patients and is highly correlated with plasmocyte tumor burden and genetic aberrations. For example, the GPRC5D in the present application may refer specifically to GPRC5D expressed in MM patients.

In the present application, the term "IgG" refers to a polypeptide belonging to a class of antibodies that are substantially encoded by the recognized immunoglobulin γ gene. In humans, this class comprise IgG1, IgG2, IgG3, and IgG4. In mice, this class comprises IgG1, IgG2a, IgG2b, and IgG3.

In the present application, the term "chimeric antigen receptor" (CAR) generally refers to a recombinant peptide that comprises at least an extracellular domain, a transmembrane domain, and an intracellular domain, which bind specifically to an antigen or target. For example, a hinge region is included between the extracellular domain and the transmembrane region. For example, the chimeric antigen receptor may further comprise a low-density lipoprotein receptor-related protein or a fragment thereof. For example, the chimeric antigen receptor may comprise a signal peptide. The binding of the extracellular domain of the CAR to a target antigen on the surface of a target cell leads to the clustering of the CAR and the delivery of an activation stimulus to a CAR-containing cell. CAR redirects the specificity of immune effector cells, and triggers proliferation, cytokine production, and the phagocytosis and/or production of molecules capable of mediating the death of target antigen-expressing cells in a major histocompatibility (MHC)-independent manner. For example, the extracellular structure may comprise the antigen binding protein defined above. For example, the extracellular structure may specifically bind to GPRC5D.

In the present application, the term "intracellular domain" means an intracellular domain that includes any truncated moiety sufficient to transduce an activation signal. The intracellular domain may comprise an intracellular signal region and/or a co-stimulatory signal region. The term "intracellular signal region" refers to an intracellular region capable of producing signals that promote the function of immune effectors of CAR-containing cells (for example, CART cells or CAR-expressing NK cells). For example, the intracellular signal region may comprise an intracellular signal region of one or more proteins selected from the group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj 14 Nef, Kaposi sarcoma-associated herpesvirus (HSKV), DAP 10, DAP-12 and a domain comprising at least one ITAM domain. For example, the intracellular signal region may be a CD3ζ-derived signaling domain. The term "co-stimulatory signal region" refers to a moiety of the CAR capable of transducing an effector signal, in the intracellular signal region. For example, the co-stimulatory signal region may comprise an intracellular co-stimulatory signal region derived from one or more proteins selected from the group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD83 ligands, CD40, and MyD88. For example, the co-stimulatory signal region may be a 4-1BB-derived intracellular co-stimulatory signal region.

In the present application, the term "transmembrane region" refers to the domain of a peptide, polypeptide, or protein capable of crossing the cytoplasmic membrane. These domains can be used to anchor the extracellular domains to cytomembranes. For example, the transmembrane region may comprise a transmembrane domain of one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154 and SLAM. For example, the transmembrane region may be a CD8-derived transmembrane region.

In the present application, the term "hinge region" denotes a moiety of the antibody heavy-chain polypeptide for connecting the CH1 and CH2 domains, for example, the moiety from about position 216 to position 230 in the EU numbering system according to Kabat. The hinge region is generally a dimer molecule consisting of two polypeptides with the same amino acid sequence. The hinge region generally comprises about 25 amino acid residues and is flexible, allowing the antigen binding region to move independently. The hinge region may be subdivided into 3 domains, including: upper, middle, and lower hinge domains. For example, the hinge region may comprise a hinge region derived from one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30 and LIGHT. For example, the hinge region may be a CD8-derived hinge region.

In the present application, the term "low-density lipoprotein receptor-related protein" refers to a cell surface protein pertaining to an endocytic receptor. It is widely distributed in organisms and greatly varies among tissues, with the main function of taking cholesterol into cells for cell proliferation and the synthesis of sterol hormones and bile salts. For example, the low-density lipoprotein receptor-related protein may be sourced from any vertebrate. For example, the low-density lipoprotein receptor-related protein or the fragment thereof may be located at the C-terminal of the intracellular signal region. For example, the low-density lipoprotein receptor-related protein or the fragment thereof may comprise one or more selected from the group consisting of: low-density lipoprotein receptor-related proteins 1-12 or fragments thereof. For example, the low-density lipoprotein receptor-related protein or the fragment thereof may be a low-density lipoprotein receptor-related protein 6 or a fragment thereof.

In the present application, the term "signal peptide" refers to a leader sequence at the amino terminal (N-terminal) of a newborn CAR protein. It guides the newborn protein to the endoplasmic reticulum during or after translation, for subsequent surface expression. For example, the signal peptide is a CD8 protein-derived signal peptide. For example, the signal peptide comprises an amino acid sequence as set forth in SEQ ID NO: 72.

In the present application, the terms "polypeptide", "peptide" and "protein" are used interchangeably herein and refer to polymers of amino acid residues. These terms may be used to indicate amino acid polymers in which one or more amino acid residues are synthetic chemical mimics of their corresponding natural amino acids, and also to indicate natural amino acid polymers, amino acid polymers containing modified residues, and unnatural amino acid polymers. For example, the peptide may comprise the antigen-binding protein.

In the present application, the term "nucleic acid molecule" comprises DNA molecules and RNA molecules. A nucleic acid molecule may be single-stranded or double-stranded, but preferably a double-stranded DNA. The term "promoter" generally refers to a DNA sequence. It can regulate the expression of a selected DNA sequence operably linked to the promoter, thereby affecting the expression of the selected DNA sequence in the cell. For example, the nucleic acid molecule may encode the antigen binding protein and/or the chimeric antigen receptor. For example, the nucleic acid molecule may comprise a promoter. For example, the promoter may be a constitutive promoter. For example, the promoter may be an EF1$\alpha$ promoter.

In the present application, the term "vector" generally refers to a molecule to which one or more nucleic acid molecules of the present application can be attached. For example, the vector may be a viral vector. For example, the vector may be a lentiviral vector.

In the present application, the term "cell" refers to a cell to which a nucleic acid can be transfected, and the term "cell" comprises a prokaryotic cell for plasmid propagation, and an eukaryotic cell for nucleic acid expression and encoding peptide production. For example, the cell may comprise the antigen binding protein, the nucleic acid molecule, and/or the vector. For example, the cell may be an immune effector cell. The term "immune effector cell" generally refers to an immune cell that participates in immune responses and exerts an effector function. For example, exerting the effector function may comprise removing xenoantigens, promoting immune effector responses or the like. For example, the immune effector cell may comprise a T cell, a B cell, a natural killer (NK) cell, a macrophage, a NKT cell, a monocyte, a dendritic cell, a granulocyte, a lymphocyte, a leukocyte, a peripheral blood mononuclear cell, an embryonic stem cell, a lymphoprogenitor cell, and/or a pluripotent stem cell. For example, the immune effector cell may be a T cell.

In the present application, the term "pharmaceutical composition" generally refers to a chemical or biological composition suitable for administration to individual mammals. For example, the pharmaceutical composition may comprise the antigen binding protein, the chimeric antigen receptor, the polypeptide, the nucleic acid molecule, the carrier and/or the cell, and optionally a pharmaceutically acceptable carrier. The pharmaceutical composition may be used to prevent, treat and/or relieve diseases or disorders associated with abnormal expression of GPRC5D. For example, the diseases or disorders associated with abnormal expression of GPRC5D may comprise tumors. For example, the tumors comprise solid tumors and/or non-solid tumors. For example, the tumors may comprise hematologic neoplasms and/or lymphomas. For example, the tumors may comprise melanomas.

## DETAILED DESCRIPTION OF THE INVENTION

Isolated Antigen Binding Protein

In one aspect, the present application provides an isolated antigen binding protein capable of binding to a G protein-coupled receptor, class C, group 5, member D (GPRC5D) protein with an EC50 value below 1.0 $\mu$g/mL. For example, the antigen binding proein of the present application may bind to the GPRC5D protein with the EC50 value below about 0.9 $\mu$g/mL, below about 0.8 $\mu$g/mL, below about 0.7 $\mu$g/mL, below about 0.6 $\mu$g/mL, below about 0.5 $\mu$g/mL, below about 0.4 $\mu$g/mL, below about 0.3 $\mu$g/mL, below about 0.2 $\mu$g/mL, below about 0.1 $\mu$g/mL or lower. For example, the antigen binding proein of the present application may bind to the cell surface-expressing GPRC5D protein with the EC50 value below about 0.9 $\mu$g/mL. For example, the binding of the antigen binding protein of the present application to the cell surface-expressing GPRC5D protein can be detected by flow cytometric fluorescence-activated cell sorting (FACS), for example, by using an iQue Screener flow cytometer.

In the present application, the antigen binding protein may comprise an antibody or an antigen binding fragment thereof. In the present application, the antigen binding fragment may comprise Fab, Fab', F(ab)$_2$, a Fv fragment, F(ab')$_2$, scFv, di-scFv, VHH and/or dAb. In the present application, the antibody may comprise a monoclonal antibody, a chimeric antibody, a humanized antibody and a fully human antibody.

CDR

CDR is the complementary determining region of an antibody, as well as a fraction of the variable region of the antibody. The amino acid residues in CDR come into contact with antigens or antigenic epitopes to specifically recognize and bind the antigens.

In the present application, the isolated antigen binding protein may comprise at least one CDR in an antibody heavy-chain variable region VH. For example, the VH may comprise an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

In the present application, the antigen binding protein may comprise HCDR3. For example, the HCDR3 of the antigen binding protein may comprise an amino acid sequence as set forth in SEQ ID NO: 215:
AAX$_3$X$_4$X$_5$X$_6$X$_7$X$_8$X$_9$X$_{10}$X$_{11}$X$_{12}$X$_{13}$X$_{14}$YX$_{16}$Y (SEQ ID NO: 215), with X$_3$ = F, G, R, S, T or V, X$_4$ = A, G, L, P, R, S or T, X$_5$ = A, G, K, R, V or Y, X$_6$ = P, R, S, V or Y, X$_7$ = A, F, G, I, N, P, S, W or Y, X$_8$ = A, F, L, P, R, T or Y, X$_9$ = A, G, L, R, S, T or Y, X$_{10}$ = L, P, Q, S, T, W or Y, X$_{11}$ = D, G, N, R, S, T or Y, X$_{12}$ = A, D, E, G, P, R, S or Y, X$_{13}$ = A, G, L, N, R, S or Y, X$_{14}$ = E, G, L, N, R, T or V, and X$_{16}$= D or N. For example, the sequence may be divided according to the IMGT rules.

In the present application, the antigen binding protein may comprise HCDR3. For example, the HCDR3 of the antigen binding protein may comprise an amino acid sequence as set forth in SEQ ID NO: 218:
AAX$_3$X$_4$X$_5$X$_6$X$_7$X$_8$X$_9$X$_{10}$X$_{11}$YX$_{13}$Y (SEQ ID NO: 218), with X$_3$ = D, F, G, K, N, R, S, T or V, X$_4$ = D, G, I, L, R, S or T, X$_5$ = A, F, G, R, V or Y, X$_6$ = G, P, R, S, T, W or Y, X$_7$ = A, G, L, N, R, S, W or Y, X$_8$ = D, F, G, L, N, R, S, T or Y, X$_9$ = A, D, G, I, L, P, R, S, T or Y, X$_{10}$ = G, L, N, R, S, T, W or Y, X$_{11}$ = D, G, L, N, P, R or T, and X$_{13}$ = D or N.

In the present application, the antigen binding protein may comprise HCDR3, and the HCDR3 may comprise an amino acid sequence as set forth in any one of SEQ ID NO: 97, SEQ ID NO: 104, SEQ ID NO: 107, SEQ ID NO: 111, SEQ ID NO: 115, SEQ ID NO: 118, SEQ ID NO: 121, SEQ ID NO: 125, SEQ ID NO: 129, SEQ ID NO: 132, SEQ ID NO: 135, SEQ ID NO: 137, SEQ ID NO: 140, SEQ ID NO: 144, SEQ ID NO: 147, SEQ ID NO: 150, SEQ ID NO: 154, SEQ ID NO: 156, SEQ ID NO: 158, SEQ ID NO: 161, SEQ ID NO: 163, SEQ ID NO: 165, SEQ ID NO: 168, SEQ ID NO: 170, SEQ ID NO: 172, SEQ ID NO: 176, SEQ ID NO: 179, SEQ ID NO: 182, SEQ ID NO: 185, SEQ ID NO: 188, SEQ ID NO: 191, SEQ ID NO: 195 and SEQ ID NO: 198.

In the present application, the antigen binding protein may comprise HCDR2. For example, the HCDR2 of the antigen binding protein may comprise an amino acid sequence as set forth below:
X$_1$X$_2$X$_3$X$_4$X$_5$X$_6$X$_7$T, with X$_1$ = I or T, X$_2$ = N, S or T, X$_3$ = A, P, R, S or W, X$_4$ = G, R, S, T or none, X$_5$ = D or G, X$_6$ = G or S, and X$_7$ = D, G, I, N, R, S, T or V For example, the sequence may be divided according to the IMGT rules.

In the present application, the antigen binding protein may comprise HCDR2, and the HCDR2 may comprise an amino acid sequence as set forth in any one of SEQ ID NO: 96, SEQ ID NO: 103, SEQ ID NO: 106, SEQ ID NO: 110, SEQ ID NO: 114, SEQ ID NO: 117, SEQ ID NO: 120, SEQ ID NO: 124, SEQ ID NO: 128, SEQ ID NO: 131, SEQ ID NO: 134, SEQ ID NO: 139, SEQ ID NO: 143, SEQ ID NO: 146, SEQ ID NO: 149, SEQ ID NO: 153, SEQ ID NO: 155, SEQ ID NO: 160, SEQ ID NO: 162, SEQ ID NO: 164, SEQ ID NO: 167, SEQ ID NO: 171, SEQ ID NO: 175, SEQ ID NO: 178, SEQ ID NO: 181, SEQ ID NO: 187, SEQ ID NO: 190, SEQ ID NO: 192, SEQ ID NO: 194 and SEQ ID NO: 197.

In the present application, the antigen binding protein may comprise HCDR1. For example, the HCDR1 of the antigen binding protein may comprise an amino acid sequence as set forth below:
GX$_2$X$_3$X$_4$SX$_6$X$_7$X$_8$, with X$_2$ = F, G, I, L, N, R, S or Y, X$_3$ = I or T, X$_4$ = F or L, X$_6$ = I, L, N, R, S, T or Y, X$_7$= D, N or Y, and X$_8$= A, D, G, I, N, R, S, T, V or Y For example, the sequence may be divided according to the IMGT rules.

In the present application, the antigen binding protein may comprise HCDR1, and the HCDR1 may comprise an amino acid sequence as set forth in any one of SEQ ID NO: 95, SEQ ID NO: 102, SEQ ID NO: 105, SEQ ID NO: 109, SEQ ID NO: 116, SEQ ID NO: 119, SEQ ID NO: 123, SEQ ID NO: 127, SEQ ID NO: 130, SEQ ID NO: 133, SEQ ID NO: 136, SEQ ID NO: 138, SEQ ID NO: 142, SEQ ID NO: 145, SEQ ID NO: 148, SEQ ID NO: 152, SEQ ID NO: 157, SEQ ID NO: 159, SEQ ID NO: 166, SEQ ID NO: 169, SEQ ID NO: 174, SEQ ID NO: 177, SEQ ID NO: 180, SEQ ID NO: 184, SEQ ID NO: 186, SEQ ID NO: 189, SEQ ID NO: 193 and SEQ ID NO: 196.

In the present application, the antigen binding protein may comprise HCDR1, HCDR2, and HCDR3, the HCDR1 may comprise an amino acid sequence as set forth in any one of SEQ ID NO: 95, SEQ ID NO: 102, SEQ ID NO: 105, SEQ ID NO: 109, SEQ ID NO: 116, SEQ ID NO: 119, SEQ ID NO: 123, SEQ ID NO: 127, SEQ ID NO: 130, SEQ ID NO: 133, SEQ ID NO: 136, SEQ ID NO: 138, SEQ ID NO: 142, SEQ ID NO: 145, SEQ ID NO: 148, SEQ ID NO: 152, SEQ ID NO: 157, SEQ ID NO: 159, SEQ ID NO: 166, SEQ ID NO: 169, SEQ ID NO: 174, SEQ ID NO: 177, SEQ ID NO: 180, SEQ ID NO: 184, SEQ ID NO: 186, SEQ ID NO: 189, SEQ ID NO: 193 and SEQ ID NO: 196, the HCDR2 may comprise an amino acid sequence as set forth in any one of SEQ ID NO: 96, SEQ ID NO: 103, SEQ ID NO: 106, SEQ ID NO: 110, SEQ ID NO: 114, SEQ ID NO: 117, SEQ ID NO: 120, SEQ ID NO: 124, SEQ ID NO: 128, SEQ ID NO: 131, SEQ ID NO: 134, SEQ ID NO: 139, SEQ ID NO: 143, SEQ ID NO: 146, SEQ ID NO: 149, SEQ ID NO: 153, SEQ ID NO: 155, SEQ ID NO: 160, SEQ ID NO: 162, SEQ ID NO: 164, SEQ ID NO: 167, SEQ ID NO: 171, SEQ ID NO: 175, SEQ ID NO: 178, SEQ ID NO: 181, SEQ ID NO: 187, SEQ ID NO: 190, SEQ ID NO: 192, SEQ ID NO: 194 and SEQ ID NO.: 197, and the HCDR3 may comprise an amino acid sequence as set forth in any one of SEQ ID NO: 97, SEQ ID NO: 104, SEQ ID NO: 107, SEQ ID NO: 111, SEQ ID NO: 115, SEQ ID NO: 118, SEQ ID NO: 121, SEQ ID NO: 125, SEQ ID NO: 129, SEQ ID NO: 132, SEQ ID NO: 135, SEQ ID NO: 137, SEQ ID NO: 140, SEQ ID NO: 144, SEQ ID NO: 147, SEQ ID NO: 150, SEQ ID NO: 154, SEQ ID NO: 156, SEQ ID NO: 158, SEQ ID NO: 161, SEQ ID NO: 163, SEQ ID NO: 165, SEQ ID NO: 168, SEQ ID NO: 170, SEQ ID NO: 172, SEQ ID NO: 176, SEQ ID NO: 179, SEQ ID NO: 182, SEQ ID NO: 185, SEQ ID NO: 188, SEQ ID NO: 191, SEQ ID NO: 195 and SEQ ID NO: 198.

In the present application, the antigen binding protein may comprise HCDR1, HCDR2, and HCDR3, and the HCDR1, HCDR2, and HCDR3 may comprise any set of amino acid sequences selected from the group consisting of:

(1) HCDR1: SEQ ID NO: 102, HCDR2: SEQ ID NO: 103, and HCDR3: SEQ ID NO: 104;

(2) HCDR1: SEQ ID NO: 105, HCDR2: SEQ ID NO: 106, and HCDR3: SEQ ID NO: 107;

(3) HCDR1: SEQ ID NO: 109, HCDR2: SEQ ID NO: 110, and HCDR3: SEQ ID NO: 111;

(4) HCDR1: SEQ ID NO: 105, HCDR2: SEQ ID NO: 114, and HCDR3: SEQ ID NO: 115;

(5) HCDR1: SEQ ID NO: 116, HCDR2: SEQ ID NO: 117, and HCDR3: SEQ ID NO: 118;

(6) HCDR1: SEQ ID NO: 119, HCDR2: SEQ ID NO: 120, and HCDR3: SEQ ID NO: 121;

(7) HCDR1: SEQ ID NO: 123, HCDR2: SEQ ID NO: 124, and HCDR3: SEQ ID NO: 125;

(8) HCDR1: SEQ ID NO: 127, HCDR2: SEQ ID NO: 128, and HCDR3: SEQ ID NO: 129;

(9) HCDR1: SEQ ID NO: 130, HCDR2: SEQ ID NO: 131, and HCDR3: SEQ ID NO: 132;

(10) HCDR1: SEQ ID NO: 95, HCDR2: SEQ ID NO: 96, and HCDR3: SEQ ID NO: 97;

(11) HCDR1: SEQ ID NO: 133, HCDR2: SEQ ID NO: 134, and HCDR3: SEQ ID NO: 135;

(12) HCDR1: SEQ ID NO: 136, HCDR2: SEQ ID NO: 134, and HCDR3: SEQ ID NO: 137;

(13) HCDR1: SEQ ID NO: 138, HCDR2: SEQ ID NO: 139, and HCDR3: SEQ ID NO: 140;

(14) HCDR1: SEQ ID NO: 142, HCDR2: SEQ ID NO: 143, and HCDR3: SEQ ID NO: 144;

(15) HCDR1: SEQ ID NO: 145, HCDR2: SEQ ID NO: 146, and HCDR3: SEQ ID NO: 147;

(16) HCDR1: SEQ ID NO: 148, HCDR2: SEQ ID NO: 149, and HCDR3: SEQ ID NO: 150;

(17) HCDR1: SEQ ID NO: 152, HCDR2: SEQ ID NO: 153, and HCDR3: SEQ ID NO: 154;

(18) HCDR1: SEQ ID NO: 123, HCDR2: SEQ ID NO: 155, and HCDR3: SEQ ID NO: 156;

(19) HCDR1: SEQ ID NO: 157, HCDR2: SEQ ID NO: 153, and HCDR3: SEQ ID NO: 158;

(20) HCDR1: SEQ ID NO: 159, HCDR2: SEQ ID NO: 160, and HCDR3: SEQ ID NO: 161;

(21) HCDR1: SEQ ID NO: 159, HCDR2: SEQ ID NO: 162, and HCDR3: SEQ ID NO: 163;

(22) HCDR1: SEQ ID NO: 159, HCDR2: SEQ ID NO: 164, and HCDR3: SEQ ID NO: 165;

(23) HCDR1: SEQ ID NO: 166, HCDR2: SEQ ID NO: 167, and HCDR3: SEQ ID NO: 168;

(24) HCDR1: SEQ ID NO: 169, HCDR2: SEQ ID NO: 153, and HCDR3: SEQ ID NO: 170;

(25) HCDR1: SEQ ID NO: 157, HCDR2: SEQ ID NO: 171, and HCDR3: SEQ ID NO: 172;

(26) HCDR1: SEQ ID NO: 174, HCDR2: SEQ ID NO: 175, and HCDR3: SEQ ID NO: 176;

(27) HCDR1: SEQ ID NO: 177, HCDR2: SEQ ID NO: 178, and HCDR3: SEQ ID NO: 179;

(28) HCDR1: SEQ ID NO: 180, HCDR2: SEQ ID NO: 181, and HCDR3: SEQ ID NO: 182;

(29) HCDR1: SEQ ID NO: 184, HCDR2: SEQ ID NO: 106, and HCDR3: SEQ ID NO: 185;

(30) HCDR1: SEQ ID NO: 186, HCDR2: SEQ ID NO: 187, and HCDR3: SEQ ID NO: 188;

(31) HCDR1: SEQ ID NO: 189, HCDR2: SEQ ID NO: 190, and HCDR3: SEQ ID NO: 191;

(32) HCDR1: SEQ ID NO: 159, HCDR2: SEQ ID NO: 192, and HCDR3: SEQ ID NO: 179;

(33) HCDR1: SEQ ID NO: 193, HCDR2: SEQ ID NO: 194, and HCDR3: SEQ ID NO: 195; and

(34) HCDR1: SEQ ID NO: 196, HCDR2: SEQ ID NO: 197, and HCDR3: SEQ ID NO: 198.

FR

In the present application, the antibody framework region FR refers to a moiety existing between more divergent (i.e., hypervariable) CDRs, in the antibody variable region. Such framework regions are typically referred to as frameworks 1 to 4 (FR1, FR2, FR3, and FR4) and provide a backbone for rendering six CDRs (three from heavy chains and three from light chains) in three-dimensional space, so as to form an antigen-binding surface.

In the present application, the antigen binding protein may comprise H-FR1, which may comprise an amino acid sequence as set forth in SEQ ID NO: 98.

In the present application, the antigen binding protein may comprise H-FR2, which may comprise an amino acid sequence as set forth in SEQ ID NO: 216: MGWFRQAPGKGRELVAX$_{17}$ (SEQ ID NO: 216), with X$_{17}$= A, F, G, R, S or V For example, the sequence may be divided according to the IMGT rules.

In the present application, the antigen binding protein may comprise H-FR2, which may comprise an amino acid sequence as set forth in any one of SEQ ID NO: 99, SEQ ID NO: 108, SEQ ID NO: 112, SEQ ID NO: 122, SEQ ID NO: 151, and SEQ ID NO: 173.

In the present application, the antigen binding protein may comprise H-FR3, which may comprise an amino acid sequence as set forth in SEQ ID NO: 217: X$_1$YPDSVEGRFTISRDNAKRMVYLQMNSLRAEDTAVYYC (SEQ ID NO: 217), with X$_1$ = H, F, S, N or Y. For example, the sequence may be divided according to the IMGT rules.

In the present application, the antigen binding protein may comprise H-FR3, which may comprise an amino acid sequence as set forth in any one of SEQ ID NO: 100, SEQ ID NO: 113, SEQ ID NO: 126, SEQ ID NO: 141, and SEQ ID NO: 183.

In the present application, the antigen binding protein may comprise H-FR4, which may comprise an amino acid sequence as set forth in SEQ ID NO: 101.

In the present application, the antigen binding protein may comprise H-FR1, H-FR2, H-FR3 and H-FR4, which may sequentially comprise the amino acid sequences as set forth in SEQ ID NO: 98, SEQ ID NO: 216, SEQ ID NO: 217, and SEQ ID NO: 101.

In the present application, the antigen binding protein may comprise H-FR1, H-FR2, H-FR3 and H-FR4, the H-FR1 may comprise an amino acid sequence as set forth in SEQ ID NO: 98, the H-FR2 may comprise an amino acid sequence as set forth in any one of SEQ ID NO: 99, SEQ ID NO: 108, SEQ ID NO: 112, SEQ ID NO: 122, SEQ ID NO: 151, and SEQ ID NO: 173, the H-FR3 may comprise an amino acid sequence as set forth in any one of SEQ ID NO: 100, SEQ ID NO: 113, SEQ ID NO: 126, SEQ ID NO: 141, and SEQ ID NO: 183, and the H-FR4 may comprise an amino acid sequence as set forth in SEQ ID NO: 101.

In the present application, the antigen binding protein may comprise an antibody heavy-chain variable region VH, and the VH may comprise an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

In the present application, the antigen binding protein may be VHH, and the VHH may comprise an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

Heavy-chain Constant Region

In the present application, the isolated antigen binding protein may comprise a heavy-chain constant region. The heavy-chain constant region refers to a region comprising at least three heavy-chain constant domains CH1, CH2, and CH3. The non-limiting exemplary heavy-chain constant regions comprise $\gamma$, $\delta$, and $\alpha$. The non-limiting exemplary heavy-chain constant regions further comprise $\epsilon$ and $\mu$. Each heavy-chain constant region corresponds to one antibody isoform. For example, an antibody comprising the $\gamma$ constant region is an IgG antibody, an antibody comprising the $\delta$ constant region is an IgD antibody, and an antibody comprising the $\alpha$ constant region is an IgA antibody. Furthermore, an antibody comprising the $\mu$ constant region is an IgM antibody, and an antibody comprising a $\epsilon$ constant region is an IgE antibody. Some isoforms may be further subdivided into subclasses. For example, the IgG antibody includes, but is not limited to, IgG1 (comprising a $\gamma_1$ constant region), IgG2 (comprising a $\gamma_2$ constant region), IgG3 (comprising a $\gamma_3$ constant region), and IgG4 (comprising a $\gamma_4$ constant region) antibodies; the IgA antibody includes, but is not limited to, IgA1 (comprising an $\alpha_1$ constant region) and IgA2 (comprising an $\alpha_2$ constant region) antibodies; and IgM includes, but is not limited to, IgM1 and IgM2.

In the present application, the antigen binding protein may comprise an antibody heavy-chain constant region, which may be derived from IgG. In the present application, the antigen binding protein may comprises an antibody heavy-chain constant region, which may be derived from human IgG. In the present application, the antigen binding protein may comprise an antibody heavy-chain constant region, which may be derived from human IgG1.

Chimeric Antigen Receptor

In another aspect, the present application further provides a chimeric antigen receptor (CAR), which may comprise a targeting moiety binding to a GPRC5D protein. For example, the targeting moiety binding to the GPRC5D protein may be the antigen binding protein of the present application.

For example, the CAR of the present application may comprise VHH, and the VHH may comprise an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

In the present application, the CAR comprises an extracellular targeted moiety binding to the GPRC5D protein and may further comprise an intracellular domain.

In the present application, the CAR may comprise an intracellular co-stimulatory signal region, which may provide a stimulatory signal. For example, the co-stimulatory signal region may comprise an intracellular co-stimulatory signal region of one or more proteins selected from the group consisting of: CD28, 4-1BB, CD27, CD2, CD7, CD8, OX40, CD226, DR3, SLAM, CDS, ICAM-1, NKG2D, NKG2C, B7-H3, 2B4, Fc$\epsilon$RI$\gamma$, BTLA, GITR, HVEM, DAP10, DAP12, CD30, CD40, CD40L, TIM1, PD-1, LFA-1, LIGHT, JAML, CD244, CD100, ICOS, CD83 ligands, CD40, and MyD88.

For example, the co-stimulatory signal region may be a 4-1BB-derived intracellular co-stimulatory signal region. For example, the co-stimulatory signal region may comprise an amino acid sequence as set forth in SEQ ID NO: 78.

In some instances, the CAR may comprise an intracellular signal region, which may comprise a domain with at least one ITAM motif. The intracellular signaling domain may transmit a stimulatory signal into a cell. For example, the intracellular signal region may comprise an intracellular signal region derived from one or more proteins selected from the group consisting of: CD3$\zeta$, CD3$\delta$, CD3$\gamma$, CD3$\epsilon$, CD79a, CD79b, Fc$\epsilon$RI$\gamma$, Fc$\epsilon$RI$\beta$, FcyRIIa, bovine leukemia virus gp30, Epstein-Barr virus (EBV) LMP2A, simian immunodeficiency virus PBj 14 Nef, Kaposi sarcoma-associated herpesvirus (HSKV), DAP10, DAP-12 and other domains comprising at least one ITAM domain.

For example, the intracellular signal region may be a CD3ζ-derived signaling domain. For example, the intracellular signal region may comprise an amino acid sequence as set forth in SEQ ID NO: 80.

In some instances, the CAR may comprise a transmembrane domain, which is a transmembrane sequence in a cell surface protein and which may comprise a hydrophobic alpha helix. The transmembrane domain may be derived from any type I transmembrane protein. The transmembrane domain may be a synthetic sequence predicted to form a hydrophobic helix. For example, the transmembrane region may comprise a transmembrane domain derived from one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154 and SLAM.

For example, the transmembrane region may be a CD8-derived transmembrane region. For example, the transmembrane region may comprise an amino acid sequence as set forth in SEQ ID NO: 76.

In some instances, the CAR may comprise a hinge region, which may be located between the extracellular targeted moiety and the transmembrane domain. For example, the hinge region may comprise a hinge region of one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30 and LIGHT.

For example, the hinge region may be a CD8-derived hinge region. For example, the hinge region may comprise an amino acid sequence as set forth in SEQ ID NO: 74.

In the present application, at the N-terminal of the targeted moiety binding to the GPRC5D protein, the CAR may further comprise a signal peptide. For example, the signal peptide may be a CD8 protein-derived signal peptide. For example, the signal peptide may comprise an amino acid sequence as set forth in SEQ ID NO: 72.

In the present application, the CAR may further comprise a low-density lipoprotein receptor-related protein or a fragment thereof. For example, the low-density lipoprotein receptor-related protein or the fragment thereof may be located at the C-terminal of the CAR. For example, the low-density lipoprotein receptor-related protein or the fragment thereof may comprise low-density lipoprotein receptor-related proteins 1-12 or fragments thereof. For example, the low-density lipoprotein receptor-related protein or the fragment thereof may be a low-density lipoprotein receptor-related protein 6 or a fragment thereof. For example, the low-density lipoprotein receptor-related protein or the fragment thereof may comprise an amino acid sequence as set forth in SEQ ID NO: 84.

In the present application, the low-density lipoprotein receptor-related protein or the fragment thereof in the CAR may be linked to the C-terminal of the CAR via a self-cleaving peptide (for example, T2A, P2A, E2A or other 2A peptides). For example, the low-density lipoprotein receptor-related protein or the fragment thereof may be linked to the C-terminal of the intracellular signal region via T2A.

In the present application, from the N-terminal to the C-terminal, the CAR may sequentially comprise a targeted moiety (for example, the antigen binding protein, for another example, the VHH of the present application) binding to the GPRC5D protein, the hinge region, the transmembrane domain, the co-stimulatory signal region, and the intracellular signal region. For example, from the N-terminal to the C-terminal, the CAR may sequentially comprise the VHH, the CD8-derived hinge region, the CD8-derived transmembrane region, the 4-1BB-derived co-stimulatory signal region, and the CD3ζ-derived intracellular signal region, and the VHH may comprise an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

In the present application, from the N-terminal to the C-terminal, the CAR may sequentially comprise a targeted moiety (for example, the antigen binding protein, for another example, the VHH of the present application) binding to the CPRC50D protein, the hinge region, the transmembrane domain, the co-stimulatory signal region, the intracellular signal region, and the low-density lipoprotein antibody-related protein or a fragment thereof. For example, from the N-terminal to the C-terminal, the CAR may sequentially comprise the VHH, the CD8-derived hinge region, the CD8-derived transmembrane region, the 4-1BB-derived co-stimulatory signal region, the CD3ζ-derived intracellular signal region, and the low-density lipoprotein antibody-related protein comprising an amino acid sequence as set forth in SEQ ID NO: 84 or a fragment thereof, and the VHH may comprise an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

In the present application, from the N-terminal to the C-terminal, the CAR may sequentially comprise a signal peptide, a targeted moiety (for example, the antigen binding protein, for another example, the VHH of the present application) binding to the GPRC5D protein, the hinge region, the transmembrane domain, the co-stimulatory signal region, and the intracellular signal region.

In the present application, from the N-terminal to the C-terminal, the CAR may sequentially comprise a signal peptide, a targeted moiety (for example, the antigen binding protein, for another example, the VHH of the present application) binding to the GPRC5D protein, the hinge region, the transmembrane domain, the co-stimulatory signal region, the intracellular signal region, and the low-density lipoprotein antibody-related protein or a fragment thereof.

For example, the chimeric antigen receptor may comprise an amino acid sequence as set forth in any one of SEQ ID NO: 86, SEQ ID NO: 88, SEQ ID NO: 90, SEQ ID NO: 92, SEQ ID NO: 200, SEQ ID NO: 201, SEQ ID NO: 202, SEQ ID NO: 203 and SEQ ID NO: 204. For example, the chimeric antigen receptor may comprise an amino acid sequence as set forth in any one of SEQ ID NO: 205, SEQ ID NO: 206, SEQ ID NO: 207, SEQ ID NO: 208, SEQ ID NO: 209, SEQ ID NO: 210, SEQ ID NO: 211, SEQ ID NO: 212, SEQ ID NO: 213 and SEQ ID NO: 214.

For example, the chimeric antigen receptor may be encoded by a nucleotide sequence as set forth in any one of SEQ ID NO: 85, SEQ ID NO: 87, SEQ ID NO: 89, and SEQ ID NO: 91.

Nucleic Acid Molecule

In another aspect, the present application further provides an isolated nucleic acid molecule or isolated nucleic acid molecules. The nucleic acid molecule may be an isolated form of nucleotide, deoxynucleotide and/or ribonucleotide of an arbitrary length, and may encode the isolated antigen binding protein and/or the chimeric antigen receptor.

For example, the nucleic acid molecule may comprise a promoter. For example, the promoter may be a constitutive promoter. For example, the promoter may be an $EF1\alpha$ promoter.

For example, the nucleic acid molecule may comprise a nucleotide sequence as set forth in any one of SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67 and SEQ ID NO: 69. For example, the nucleic acid molecule may comprise a nucleotide sequence as set forth in any one of SEQ ID NO: 85, SEQ ID NO: 87, SEQ ID NO: 89, and SEQ ID NO: 91.

Vector

In another aspect, the present application further provides a vector, which may comprise the nucleic acid molecule. The vector may be transformed, transduced, or transfected into a host cell, such that genetic material elements carried by the vector can be expressed in the host cell. For example, the vector may comprise a promoter, a transcript, an enhancer, a replicon, a selection element, and a reporter gene. For example, the vector may comprise a component assisting in entering a cell. To allow replication of the nucleic acid molecule in the vector, the 5'-terminal and 3'-terminal of the nucleic acid molecule may further comprise long terminal repeats.

For example, the vector may be a viral vector. For example, the vector may be a lentiviral vector.

Cell

In another aspect, the present application further provides a cell. The cell may comprise the isolated antigen binding protein, the chimeric antigen receptor, the nucleic acid molecule and/or the vector. The cell may comprise the progeny of a single cell. Due to natural, accidental, or deliberate mutations, the progeny may not necessarily be exactly the same as an original parent cell (in the form of total DNA complement or in the genome).

In some embodiments, the cell may be an immune effector cell. In some embodiments, the cell may comprise a T cell, a B cell, a natural killer (NK) cell, a macrophage, a NKT cell, a monocyte, a dendritic cell, a granulocyte, a lymphocyte, a leukocyte, a peripheral blood mononuclear cell, an embryonic stem cell, a lymphoprogenitor cell, and/or a pluripotent stem cell. For example, in some embodiments, the cell may be a T cell.

Pharmaceutical Composition

In another aspect, the present application further provides a pharmaceutical composition, which may comprise the isolated antigen binding protein, the chimeric antigen receptor, the nucleic acid molecule, the vector, and/or the cell, and optionally, a pharmaceutically acceptable adjuvant.

In some embodiments, the pharmaceutical composition may further comprise a suitable formulation of one or more (pharmaceutically effective) carriers, stabilizers, excipients, diluents, solubilizers, surfactants, emulsifiers and/or preservatives. The acceptable ingredient of the composition is preferably nontoxic to a subject at a dose and concentration as used. The pharmaceutical composition of the present invention may comprise liquid, frozen, and lyophilized compositions.

In some embodiments, the pharmaceutically acceptable adjuvant may comprise any and all solvents, dispersion media, coatings, isotonic agents, and absorption retarders, that are compatible with pharmaceutical administration. Such an adjuvant is generally safe and non-toxic, and is neither biologically nor otherwise undesirable.

In some embodiments, the pharmaceutical composition may be administered parenterally, transdermally, intraluminally, intraarterially, intrathecally and/or intranasally, or may be directly injected into a tissue. For example, the pharmaceutical composition may be administered to a patient or subject by infusion or injection. In some embodiments, the pharmaceutical composition may be administered by different means, for example, by intravenous, intratumoral, intraperitoneal, subcutaneous, intramuscular, topical or intradermal administration.

Preparation Method

In another aspect, the present application further provides a method for preparing the isolated antigen binding protein and/or the chimeric antigen receptor. The method may comprise culturing the cell under a condition enabling expression of the antigen receptor and/or the chimeric antigen receptor.

The present application further provides a method for preparing a modified immune effector cell. The method may comprise introducing the vector into an immune cell.

Use

In another aspect, the present application further provides uses of the isolated antigen binding protein, the chimeric antigen receptor, the nucleic acid molecule, the vector, the cell and/or the pharmaceutical composition in preparation of a medicament for preventing, relieving and/or treating tumors.

In another aspect, the present application further provides a method for preventing, relieving and/or treating tumors. The method may comprise administering the isolated antigen binding protein, the chimeric antigen receptor, the nucleic acid molecule, the vector, the cell and/or the pharmaceutical composition to a subject in need thereof.

In another aspect, the present application further provides the isolated antigen binding protein, the chimeric antigen receptor, the nucleic acid molecule, the vector, the cell and/or the pharmaceutical composition for use in prevention, relieving and/or treatment of tumors.

In the present application, the tumors may comprise solid tumors and/or non-solid tumors.

In the present application, the tumors may comprise hematologic neoplasms and/or lymphomas.

In the present application, the tumors may comprise myelomas. In the present application, the tumors may comprise multiple myeloma.

In the present application, the subject may comprise a human or non-human animal.

In another aspect, the present application further provides a polypeptide, comprising the isolated antigen binding protein. In another aspect, the present application provides an antibody-drug conjugate, comprising the isolated antigen binding protein.

In another aspect, the present application provides a kit or doser, which comprises the isolated antigen binding protein, the chimeric antigen receptor, the nucleic acid molecule, the vector, the cell and/or the pharmaceutical composition.

Not wishing to be bound by any particular theory, the following examples are merely to illustrate the antigen binding protein, preparation method, uses and the like of the present application, and are not intended to limit the scope of the present application.

**Examples**

**Example 1. Screening of Anti-GPRC5D Antibodies by Artificially Synthetic Nanobody Library NanoOri_1.0**

296 non-repeating nanobodies in the PDB (Protein Data Bank) and their structures were first aligned to determine the mutation strategy of CDRs. Here, CDR3 had three types of lengths: 14, 17 and 21 amino acids. A commercially available nanobody, caplacizumab, was selected as the skeleton; the nucleotide sequence of caplacizumab was obtained by total gene synthesis and then cloned into an HP153 phage vector, followed by the extraction of single-stranded DNAs of HP153; and the CDRs of the nanobodies were mutated by Kunkel mutagenesis to obtain a double-stranded DNA.

The double-stranded DNA was electroporated to M13KO7-assisted phage pre-infected competent E. coli SS320, followed by overnight culture and collection of the phage supernatant, and finally, the synthetic nanobody library NanoOri_1.0 (Shanghai Origincell Medical Technology Co., Ltd.) with the diversity of $1.44 \times 10^{10}$ was constructed to serve as a seed bank for antibody sequence screening.

The GPRC5D full-length sequence (cat: HG24447-UT) purchased from Sino Biological was constructed into the company's existing lentiviral vector for lentiviral transduction to prepare overexpression cell lines of target cells CHO-GPRC5D and target cells 293-GPRC5D. The synthetic nanobody library (Shanghai Origincell Medical Technology Co., Ltd.) was panned alternately with the target cells CHO-GPRC5D and 293-GPRC5D, and a total of four rounds of panning were carried out as follows:

One nanobody from the synthetic nanobody library NanoOri_1.0 was added to PEG/NaCl and reprecipitated for later use. Target cells CHO-GPRC5D and 293-GPRC5D were selected for alternate screening of cell panning. 500 μL of phage and 500 μL of 10% FBS/PBS buffer were mixed to a final volume of 1 mL, charged into a 2 mL low-adsorption EP tube, and gently rotated or periodically blended at 4°C, followed by blockage for 1 h. Centrifugation was performed for 10 min at 140 g to harvest CHO-GPRC5D cells, which were washed once with 10 mL of PBS. The viable cells were counted till reaching more than 95%, and the CHO-GPRC5D cells were washed three times with 5% FBS/PBS buffer. Then, the CHO-GPRC5D cells were resuspended in 1 mL of 5% FBS/PBS buffer to bring the number of cells to $1 \times 10^7$, during which the cells were kept on ice. The CHO-GPRC5D cells were centrifuged for 2 min at 140 g at 4°C, resuspended in the well blocked 1 mL of phage antibody library, and gently rotated or periodically blended at 4°C or room temperature, followed by blockage for 2 hours; the supernatant was removed to harvest cells, which were resuspended in 1 mL of 5% FBS/PBS buffer, and washed twice; and after the last resuspension of the cells, the cells were added to a new 2 mL low-adsorption EP tube to prevent the phages that were not specifically adsorbed to the EP tube from being eluted.

The cells were resuspended with 1 mL of 100 nM Gly-HCl (pH 2.2), incubated for 10 min at room temperature, and then centrifuged for 2 min at 140 g at 4 °C; and the supernatant was transferred into a new EP tube, followed by the addition of 25 μL of 2 M Tris neutralization solution. About 1 mL of the neutralization solution was then neutralized with about 500 μL of 7.5% FBS/PBS buffer.

Centrifugation was performed to obtain the same 3 parts of adsorbed cell CHO, which were treated with the same method as CHO-GPRC5D cells. The eluted and neutralized phages were resuspended in the CHO cells, which were then gently rotated at 4°C, or periodically blended for 30 minutes. Centrifugation was performed for 2 min at 140 g at 4°C, and the supernatant was removed for the next adsorption process, for a total of 3 times. The last phage supernatant was used to infect TG1 cells.

The second round of cell panning was performed with the same method, where the number of target cells CHO-GPRC5D was $5 \times 10^6$ and the number of washing was 3 times. The third and fourth rounds of cell panning were performed with the same operation method, where the target cells were changed to 293-GPRC5D, the number of cells was $1 \times 10^6$, and the number of washing was increased to 10.

After 4 rounds of panning, the output phages were observed to show significant specific enrichment for the target cells. The panned phage antibody clones were identified using the cell-based monophage ELISA: the 96-well ELISA plate was coated with CHO-GPRC5D target cells at $3 \times 10^4$ cells/well overnight at 4°C. Then, the non-specific binding sites were blocked with 5% skimmed milk powder; after full washing, the monoclonal phage supernatant (premixed with the skimmed milk powder) was added to the 96-well plate, incubated for 2 hours, and fully washed, followed by the addition of anti-M13-HRP for 45-min reaction; after full washing, TMB was added for color development, the reaction was allowed to occur for 5-10 min at room temperature and then stopped with sulfuric acid; and the OD value of each well was determined at 450 nm.

34 phage antibody clones (i.e., the isolated GPRC5D antibody described in the present application) capable of specifically binding to human GPRC5D were identified by ELISA; after sequencing, single-stranded $V_HH$ gene sequences were obtained; and the 10 phage antibody clones were named as 3B5, 3E7, 4A2, 4B3, 3E9, 3F5, 3G4, 4A4, 4E12, 4F10, 505A1, 505A3, 505A11, 505B12, 505D2, 505F1, 505H3, 505H8, 505H10, 506A5, 506A8, 506B6, 506B12, 506C2, 506C5, 506C8, 506C11, 506D2, 506D8, 506E1, 506F5, 506G1, 506H2 and 506H9 respectively. The CDR sequences of the 34 clones were shown in Table 1, and the amino acid sequences in the variable region were as set forth in SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

Table 1 CDR sequences of 34 clones (divided according to IMGT)

| Clone No. | HCDR1 | HCDR2 | HCDR3 |
|-----------|-------|-------|-------|
| 3B5 | GFIFSRYG | INWSDGTT | AADRRLRSQTGYDY |

(continued)

| Clone No. | HCDR1 | HCDR2 | HCDR3 |
|---|---|---|---|
| 3E7 | GRIFSRDA | INSSGGNT | AARGVRWTSTTAYGYNY |
| 4A2 | GFIFSRYD | ITWGGGST | AARSASAYRTGRLEYNY |
| 4B3 | GLIFSRYV | INSSGGST | AASSRTSTTRGYNY |
| 3E9 | GFIFSRYD | INWGDSST | AARRYYSYYPS SGGYNY |
| 3F5 | GFILSRYV | ITWSDGST | AAKSRTYRGGLYDY |
| 3G4 | GSIFSTYR | ISPGGSDT | AATPGRFALLSPRGYNY |
| 4A4 | GFIFSRNA | INWSGSST | AAGSRTNTGTDWSRTRYTYNY |
| 4E12 | GFIFSRNS | ISWRDGST | AAFRGWNNGRNYNY |
| 4F10 | GFIFSLNA | ISPGDGTT | AARRRSLSGYGYNY |
| 505A1 | GYIFSRYN | ISAGGSST | AARRRRATD SP YD Y |
| 505A3 | GFIFSRYS | ISAGGSST | AAFARSPRTSTEYLYDY |
| 505A11 | GIIFSRYV | ITSGGGST | AAGPRPNYGQRYNTYNY |
| 505B12 | GFIFSRYI | INAGGGST | AAGSASGWSTYSPRRLYRYNY |
| 505D2 | GYIFSRYA | INRGGSTT | AAGRFGLGSRTYDY |
| 505F1 | GRTLSNNT | ISASGSRT | AAVSRVGFYTRGATYNY |
| 505H3 | GNILSINI | ISRTGGST | AASSRRSSLNTYNY |
| 505H8 | GFIFSRNA | ITWSGGTT | AARIAYSSTWTYDY |
| 505H10 | GFIFSRYY | ISRTGGST | AADLYPGFRRGYNY |
| 506A5 | GFIFSRYA | ISRGDSDT | AAVGRYYYGYYANNYNY |
| 506A8 | GFIFSRYA | INRGDSVT | AARGRTSLAYTYDY |
| 506B6 | GFIFSRYA | INAGGGTT | AAFTKRSLLTGSAVYNY |
| 506B12 | GLIFSRNA | INWGGGTT | AATTRTSRPYTYNY |
| 506C2 | GYIFSNDV | ISRTGGST | AASTRRRYATVDYRRDYTYDY |
| 506C5 | GFIFSRYY | ITSGDGST | AARSFRRDIYDYNY |
| 506C8 | GYIFSNYA | INRSGSIT | AANDVRGYYRRYNY |
| 506C11 | GLIFSSYA | INRRGGST | AAGSRSSSRLGYNY |
| 506D2 | GIIFSRYA | INRSGGRT | AAFLASWDGTTYDY |
| 506D8 | GYTLSSYY | ITWGGGST | AAARKRSVTYTTLSYAYSYDY |
| 506E1 | GYIFSRYS | ISSSGSST | AATPRRSTGNTF |
| 506F5 | GGIFSSYA | INRGGGGT | AAFGRYSRGSDDSRYNY |
| 506G1 | GFIFSRYA | ISWSGGST | AAGSRSSSRLGYNY |
| 506H2 | GRTFSYYT | TSRDGST | AAVRRWSSDSTYNY |
| 506H9 | GRTFSRYA | ISSRGSTT | AASLYVIPAWNEYGYNY |

**Example 2. Construction of Anti-GPRC5D V_HH_hFc Fusion Antibody and Its Eukaryotic Expression and Purification**

The phage antibody clones 3B5, 3E7, 4A2, 4B3, 3E9, 3F5, 3G4, 4A4, 4E12, 4F10, 505A1, 505A3, 505A11, 505B12, 505D2, 505F1, 505H3, 505H8, 505H10, 506A5, 506A8, 506B6, 506B12, 506C2, 506C5, 506C8, 506C11, 506D2, 506D8, 506E1, 506F5, 506G1, 506H2 and 506H9 and the positive control antibody ET150-5 scFv (patent CN107428829A) were redesigned and constructed into a Fc (IgG1)-containing eukaryotic expression vector: primers were designed for PCR amplification of the $V_H$Hs of the phage antibody clones 3B5, 3E7, 4A2, 4B3, 3E9, 3F5, 3G4, 4A4, 4E12, 4F10, 505A1, 505A3, 505A11, 505B12, 505D2, 505F1, 505H3, 505H8, 505H10, 506A5, 506A8, 506B6, 506B12, 506C2, 506C5, 506C8, 506C11, 506D2, 506D8, 506E1, 506F5, 506G1, 506H2 and 506H9, and the PCR products was cloned by recombination into the pcDNA3.4-hFc vector double-digested by SfiI and NotI restriction enzymes. After the sequences were found correct by sequencing, their plasmids were used to transfect 293F cells for transient expression, followed by purification by Protein A affinity column (Protein A column) to obtain 3B5, 3E7, 4A2, 4B3, 3E9, 3F5, 3G4, 4A4, 4E12, 4F10, 505A1, 505A3, 505A11, 505B12, 505D2, 505F1, 505H3, 505H8, 505H10, 506A5, 506A8, 506B6, 506B12, 506C2, 506C5, 506C8, 506C11, 506D2, 506D8, 506E1, 506F5, 506G1, 506H2 and 506H9 antibodies. The SEC purity of the antibodies was shown in Table 2.

Table 2. SEC purity of $V_H$H antibodies

| Designation | SEC Purity |
|---|---|
| 3B5 VHH-hFc | 97.5% |
| 3E7 VHH-hFc | 100% |
| 4A2 VHH-hFc | 100% |
| 4B3 VHH-hFc | 100% |
| 3E9 VHH-hFc | 76.5% |
| 3F5 VHH-hFc | 100% |
| 3G4 VHH-hFc | 100% |
| 4A4 VHH-hFc | 100% |
| 4E12VHH-hFc | 97.1% |
| 4F10 VHH-hFc | 98.7% |
| 505A1 VHH-hFc | 94.11% |
| 505A3 VHH-hFc | 93.37% |
| 506D8 VHH-hFc | 95.92% |
| 506A8 VHH-hFc | 96.52% |
| 506B12 VHH-hFc | 94.18% |
| 506C5 VHH-hFc | 90.90% |
| 506C11 VHH-hFc | 91.70% |

**Example 3. Assay on Binding Activity of Anti-GPRC5D Antibody to Cell Surface GPRC5D**

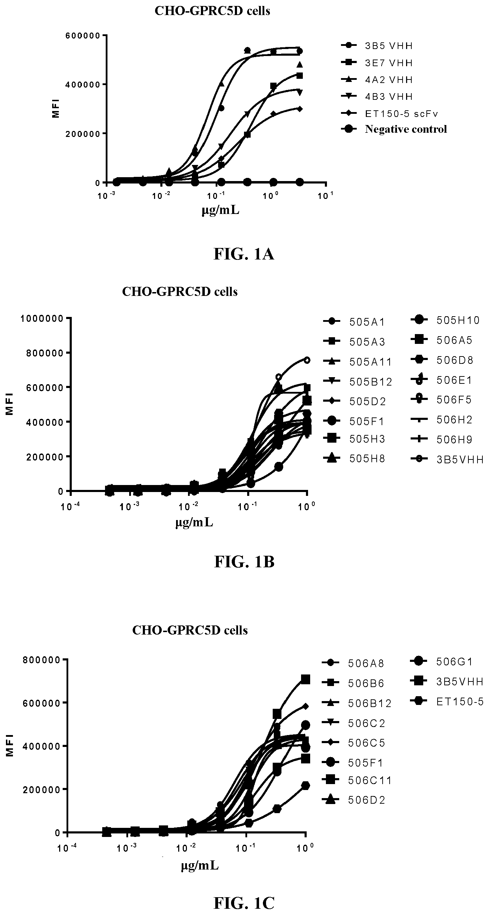

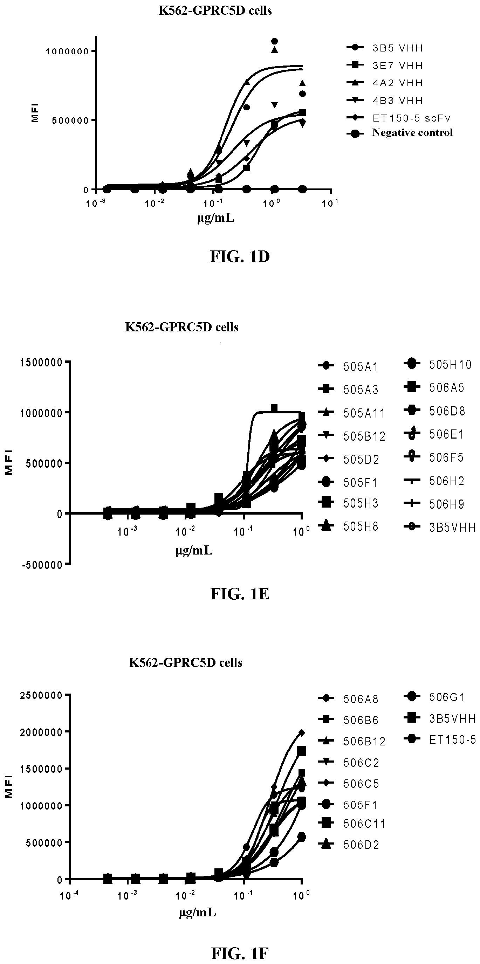

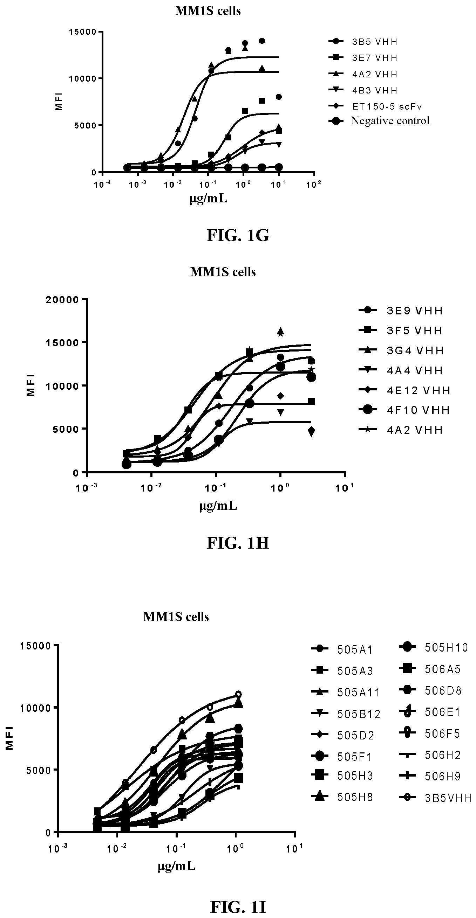

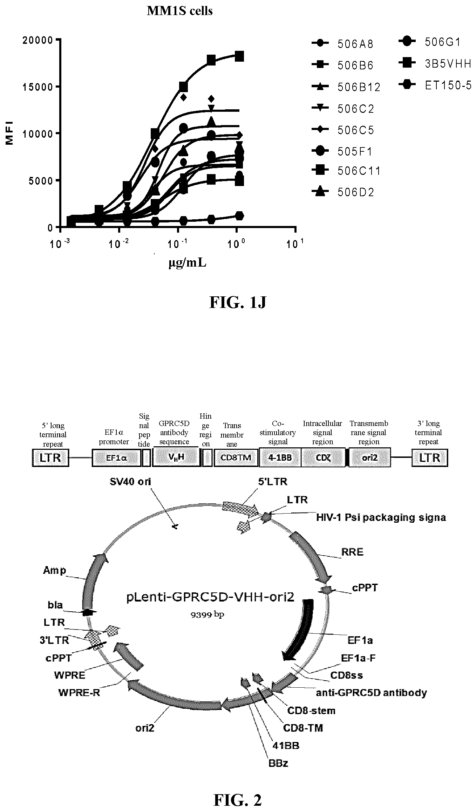

The assay on the binding activity of the target antigen (GPRC5D) on the cell surface to the antibodies (i.e., the 3B5, 3E7, 4A2, 4B3, 3E9, 3F5, 3G4, 4A4, 4E12, 4F10, 505A1, 505A3, 505A11, 505B12, 505D2, 505F1, 505H3, 505H8, 505H10, 506A5, 506A8, 506B6, 506B12, 506C2, 506C5, 506C8, 506C11, 506D2, 506D8, 506E1, 506F5, 506G1, 506H2 and 506H9 antibodies obtained in Example 2) was performed by flow cytometry fluorescence-activated cell sorting (FACS) using an iQue Screener flow cytometer (purchased from IntelliCyt) and PBS containing 0.1% BSA as buffer. Briefly, target cells (i.e., MM1S myeloma cells) with the concentration of $1 \times 10^6$ cells/mL were prepared with buffer, and added to a 96-well pointed-bottom plate (corning 3894), with 30 $\mu$L per well; the assay antibodies with the concentration of 10 $\mu$g/mL were prepared with a buffer, and diluted at a 3-fold ratio to create 8 concentration gradients; the prepared antibodies at different concentrations were added to the pre-plated target cells at 30 $\mu$L/well and mixed well; the resultant was incubated for 1 hour in a refrigerator at 4°C; 150 $\mu$L of buffer was added to each well, centrifugation was performed for 5 min at 300 g, the supernatant was discarded and the cells were loosened by shaking; washing was repeated once; fluorescent secondary antibodies (ab98593) were prepared using the buffer at a ratio of 1:200, followed by the addition of 30 $\mu$l per well to the cells and homogenous mixing, and the resultant was incubated for 30 min in the refrigerator at 4 °C; 150 $\mu$L of buffer was added to each well, centrifugation was performed for 5 min at 300 g, the supernatant was discarded, and the cells were loosened by shaking; the cells were washed twice; and 35 $\mu$L of buffer was added to each well and mixed well, followed by detection with the flow cytometer. The analysis results of the flow cytometry affinity binding assay were shown in Table 3 and FIG. 1, indicating that the anti-GPRCSD VHH of the present application can bind to GPRC5D on the cell surface, with the binding ability comparable to or even higher than that of the positive control ET150-5 scFv.

Table 3. Binding of Anti-GPRC5D Antibody to GPRC5D Protein on Cell Surface

| GPRC5D antibody (EC50 μg/mL) | Cell line | | |
|---|---|---|---|
| | CHO-GPRC5D | K562-GPRC5D | mmis |
| 3B5 VHH | 0.1035 | 0.2066 | 0.04387 |
| 3E7 VHH | 0.4259 | 0.5761 | 0.2865 |
| 4A2 VHH | 0.06866 | 0.1644 | 0.01948 |
| 4B3 VHH | 0.1805 | 0.2158 | 0.6580 |
| 505A1 VHH | 0.1055 | 0.2302 | 0.03769 |
| 505A3 VHH | 0.1163 | 0.1169 | 0.01372 |
| 505A11 VHH | 0.1255 | 0.1748 | 0.05886 |
| 505B12 VHH | 0.1546 | 0.1622 | 0.1357 |
| 505D2 VHH | 0.0886 | 0.1118 | 0.0389 |
| 505F1 VHH | 2.462 | 0.9059 | 0.6288 |
| 505H3 VHH | 0.0857 | 0.09154 | 0.3283 |
| 505H8 VHH | 0.1186 | 0.1695 | 0.04929 |
| 505H10 VHH | 0.2438 | 0.8443 | 0.06741 |
| 506A5 VHH | 0.4368 | 1.412 | 0.05372 |
| 506D8 VHH | 0.107 | 0.295 | 0.08999 |
| 506E1 VHH | 0.2181 | 1.672 | 0.04386 |
| 506F5 VHH | 0.1196 | 0.6153 | 0.06907 |
| 506H2 VHH | 0.1357 | 0.2513 | 0.2748 |
| 506H9 VHH | 0.1887 | 0.3309 | 0.2166 |
| 506A8 VHH | 0.06495 | 0.1411 | 0.03572 |
| 506B6 VHH | 0.1021 | 0.4826 | 0.07292 |
| 506B12 VHH | 0.09338 | 0.2468 | 0.07139 |
| 506C2 VHH | 0.07475 | 0.165 | 0.04642 |
| 506C5 VHH | 0.1266 | 0.2835 | 0.02748 |
| 506C8 VHH | 0.3918 | 1.743 | 0.121 |
| 506C11 VHH | 0.1451 | 0.2715 | 0.0521 |
| 506D2 VHH | 0.1264 | 0.4848 | 0.05586 |
| 506G1 VHH | 0.129 | 0.2945 | 0.02277 |
| ET150-5 scFv | 0.2490 | 0.4315 | 0.9109 |

## Example 4. Construction of Lentiviral Expression Vector for Chimeric Antigen Receptor

The CAR lentiviral core empty plasmid (self-constructed by Shanghai Origincell Medical Technology Co., Ltd., containing ori2 new elements, hereinafter referred to as the CAR lentiviral empty vector) was double-digested with SphI and NotI endonuclease (purchased from NEB) to produce an 8992 bp linearized fragments; and after gel cutting and recovery, the fragments were mixed with the VHH fragments of 3B5, 3E7, 4A2, 4B3, 3E9, 3F5, 3G4, 4A4, 4E12 and 4F10 obtained in Example 1 at a molar ratio of 1:3 for homologous recombination to transform the DH5$\alpha$ competent cells. Single colonies were picked and identified by sequencing. The elements and ligation sequences of the components of the CAR in the CAR lentiviral empty vector and the GPRC5D core plasmid (3B5VHH-CAR-ori2, 3E7-CAR-ori2, 4A2-CAR-ori2, 4B3-CAR-ori2, 3E9-CAR-ori2, 3F5-CAR-ori2, 3G4-CAR-ori2, 4A4-CAR-ori2, 4E12-CAR-ori2 and 4F10-CAR-ori2) can be found in FIG. 2 of the specification, and the amino acid sequences are set forth sequentially as in SEQ ID NO: 86, SEQ ID NO: 88, SEQ ID NO: 90, SEQ ID NO: 92, SEQ ID NO: 199, SEQ ID NO: 200, SEQ ID NO: 201, SEQ ID NO: 202, SEQ ID NO: 203 and SEQ ID NO: 204.

**Example 5. Lentivirus Packaging and Viral Titer Determination**

As an example, the lentiviral vector system for constructing the present invention belonged to the third generation, and consisted of three plasmids, namely, the packaging plasmid psPAX2 encoding the Gag-Pol protein and the Rev protein, the PMD2.G plasmid encoding the envelope protein VSV-G, and the core plasmid, i.e., the respective CAR lentiviral plasmids containing the VHH sequences in the above Example 4 (i.e., 3B5VHH-CAR-ori2, 3E7-CAR-ori2, 4A2-CAR-ori2, 4B3-CAR-ori2, 3E9-CAR-ori2, 3F5-CAR-ori2, 3G4-CAR-ori2, 4A4-CAR-ori2, 4E12-CAR-ori2 and 4F10-CAR-ori2). The expression of CAR genes in each CAR lentiviral plasmid was regulated by the elongation factor-1$\alpha$ (EF-1$\alpha$) promoter.

5.1 The lentivirus was packaged as follows:

(1) About $2.5 \times 10^6$ 293T cells with good growth status (generally, the cells within 20 passages were selected) were placed in 5 mL of 293T cell complete medium (high-glucose DMEM containing 10% FBS), mixed well, seeded in a 6 cm culture dish (to ensure that the cell confluence was 80-90% after 20-24 h), and incubated overnight at 37°C in a 5% $CO_2$ incubator.

(2) After about 20-24 h, a total of 6. 3 $\mu$g of three types of plasmids (added at the mass ratio below: CAR lentiviral core plasmid:psPAX2:PMD2.G = 4:3:2) were added to 300 $\mu$L of Opti-MEM medium and mixed well, followed by the addition of 18.9 $\mu$L of FuGENEHD transfection reagent (Promega, E2311); the resultant was carefully mixed well and allowed to stand at room temperature for 15-20 min; then the mixture was carefully added dropwise to a 6 cm culture dish, which was carefully shaken and incubated overnight in a 5% $CO_2$ incubator at 37°C.

(3) After about 20-24 h of transfection, the upper liquid in the 6 cm culture dish was carefully discarded, followed by the slow addition of 5 mL of 293T cell complete medium.

(4) After about 48 h of transfection, the upper liquid (containing dead cells and debris) in the 6 cm culture dish was collected and centrifuged for 5 min at 3000 g; the upper viral solution with cell debris removed was transferred into a new centrifuge tube, aliquoted and stored at -80°C for later use.

5.2 The lentiviral titer was detected as follows:

(1) The viral solution prepared above was quickly thawed.

(2) 293T cells with good growth status (generally, cells within 20 passages were cultured) was taken, from which the upper waste solution was discarded; the cells were washed with PBS, and digested for about 3 min at 37 °C with 0.25% trypsin (GIBICO); after the cell was completely digested, a certain volume of 293T cell complete medium was added to stop the reaction; sampling and counting were performed, and then the cell density was regulated to $2.0 \times 10^5$/mL; polybrene with the final concentration of 10 $\mu$g/mL was added; and then the cells were seeded into a six-well plate at a seeding volume of 2.5 mL per well.

(3) Then, the above viral solution was added to the six-well plate respectively. Generally, three loading gradients (2 $\mu$L, 5 $\mu$L and 10 $\mu$L) were applied to each type of viral solution, and 1-2 blank wells without the viral solution were needed as a negative control for flow cytometry.

(4) The cell culture plate was placed in a 5% $CO_2$ incubator at 37°C for statice culture.

(5) After the cells were statically cultured for 48 hours, the upper waste liquid was discarded, the cells were washed with PBS, and then digested for about 3 min at 37°C with 0.25% trypsin (GIBICO); after the cells were completely digested, a certain volume of 293T cell complete medium was added to stop the reaction; sampling and counting were performed; and then about $5 \times 10^5$ cells were added into a 1.5 mL sterile centrifuge tube and labeled.

(6) A NBS solution (a PBS solution containing 1% neonatal bovine serum) was used to make up to 1.2-1.5 mL, centrifugation was performed for 5 min at 500 g at 4°C, and the supernatant was discarded.

(7) 1 mL of NBS was added to each tube, the resultant was mixed well by gentle pipetting, centrifugation was performed for 5 min at 500 g at 4°C, and the supernatant was discarded.

(8) 50 µL of NBS and 0.8 µg of Biotin-Goat anti-human F(ab)2 antibody (109-066-006) were added to each tube, mixed well by gentle pipetting by a pipette (negative control without primary antibody), and incubated for 60 min at 4°C.

(9) After incubation, 1 mL of NBS was directly added to resuspend the cells, which was centrifuged for 5 min at 500 g at 4°C, and the supernatant was discarded.

(10) 50 µL of NBS and 0.5 µg of APC streptavidin (554067) fluorescent secondary antibody (the negative control without this protein added) were added to each tube and incubated at 4°C for 30 min.

(11) Step (9) was repeated.

(12) 200 µL of NBS was added to each tube to resuspend the cells.

(13) The percentage of fluorescence expression was detected on the BD FACS CantoII flow cytometer, followed by data processing.

The results were considered available only when the positive rate of each test tube - the positive rate of the negative control was 5%-20%.

Lentiviral titer (IM/mL) = number of plated cells × (positive rate of test tube - positive rate of control tube)/volume of seeded viral solution (mL).

The titer of each of the above VHH-containing CAR viruses was detected to be within a range of $1-10 \times 10^6$ IM/mL.

## Example 6. Infection of T cells and in vitro Amplification of BCMA CAR-T cells

The preparation method of CAR-T cells containing the GPRC5D antibody sequence and the positive control sequence 109 scFv (patent CN107428829A) was as follows:

(1) Human peripheral blood mononuclear cells (PBMCs) were isolated from blood samples collected from apheresis machines by Ficoll density gradient centrifugation (apheresis blood was provided by volunteers of the Ark Project of Shanghai Origincell Medical Technology Co., Ltd.).

(2) The PBMCs were sorted by CD3 positive magnetic beads to obtain CD3+ T cells with a purity of >90%. The specific method for sorting T cells could be found in the product specification (MACS, DS130-050-101).

(3) The CD3+ T cells were resuspended in the T cell complete medium (X-VIVO 15 (Lonza) + 5% FBS + cytokines), followed by the addition of washed CD3/CD28 Dynabeads (Gibco, 40203D) by 3 times the cell volume; then, the T cell complete medium was supplemented; the cell density was regulated to $1.0-1.2 \times 10^6$ cells/mL; and the cells were placed in a 5% $CO_2$ incubator at 37°C for activated culture (recorded as D0).

(4) Lentiviral transduction was generally performed 20-24 hours after T cell activation.

(5) The T cells activated for 20-24 h were collected, centrifuged for 5 min at 500 g, resuspended with a certain volume of T cell complete medium and then sampled and counted; according to the ratio of 2-5 for the viral multiplicity of infection (MOI), the viral solution that had been measured in titer in Example 5 was added respectively; then polybrene was added to the final concentration of 5 µg/mL, then the T cell complete medium was supplemented, the cell density was regulated to $0.6-1.0 \times 10^6$/mL, and then the cells were cultured in a 5% $CO_2$ incubator at 37 °C; and another portion of T cells without lentiviral infection was taken as a negative control group.

(6) After about 20-24 h of viral transduction, the T cells in each group were collected, and centrifuged for 5 min at 500 g; the cells were resuspended with a certain volume of T cell complete medium, and then sampled and counted; the T cell complete medium was supplemented; and the cell density was regulated to 0.5-0.7×10$^6$ cells/mL.

(7) Afterwards, the CAR-T cells were counted every 1-2 days, the T cell complete medium was supplemented according to actual situation to regulate the cell density to 0.5-1.0×10$^6$ cells/mL.

(8) In general, Dynabeads were removed on Day 7 of culture, and the total culture cycle was about 12 days.

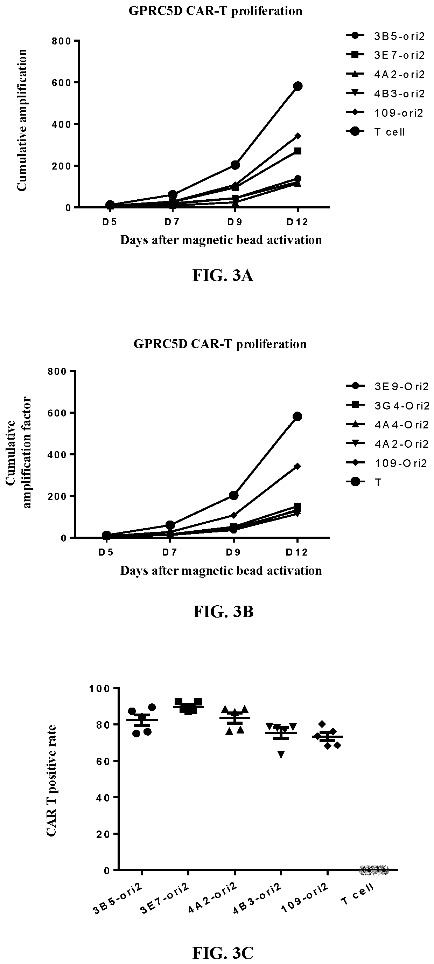

The assay results were shown in FIGs. 3A-3D, the total amplification factor of the GPRC5D CAR-T cells after 12 days of activated culture was between 100-600 folds, and the positive rate of CAR after infection was between 75% and 95%, which could be used for cytological function assays.

**Example 7. Assessment of in vitro Targeted Killing Activity of GPRC5D CAR-T Cells**

To accurately detect the killing ability of CAR-T cells against target cells, we introduced the GPRC5D gene into CHO cells by means of lentivirus, and then sorted out GPRCSD-positive cells, i.e., CHO-GPRC5D cells, by flow cytometer. The target cells were CHO-GPRC5D cells, and the negative cells were CHO-negative cells. Effector cells cultured to Day 9 were incubated with target cells for 20 hours at the effector/target ratios of 1:3, 1: 1 and 3:1, respectively, and the survival of target cells (i.e., the killing ability of CAR-T cells) was determined by an LDH method.

The killing toxicity of the cells could be calculated using the following formula:

$$\%Killing\ rate = \frac{Assay\ wells - Spontaneous\ release\ of\ target\ cells - Spontaneous\ release\ of\ effector\ cells}{Maximal\ release\ of\ target\ cells - Spontaneous\ release\ of\ target\ cells} \times 100$$

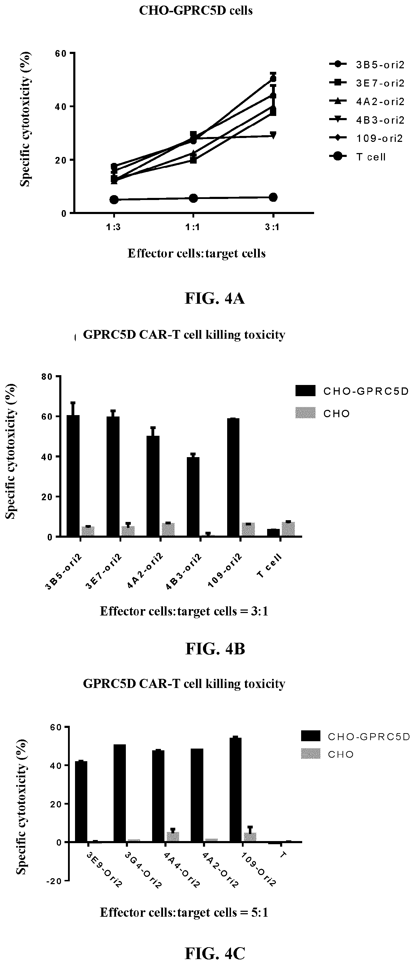

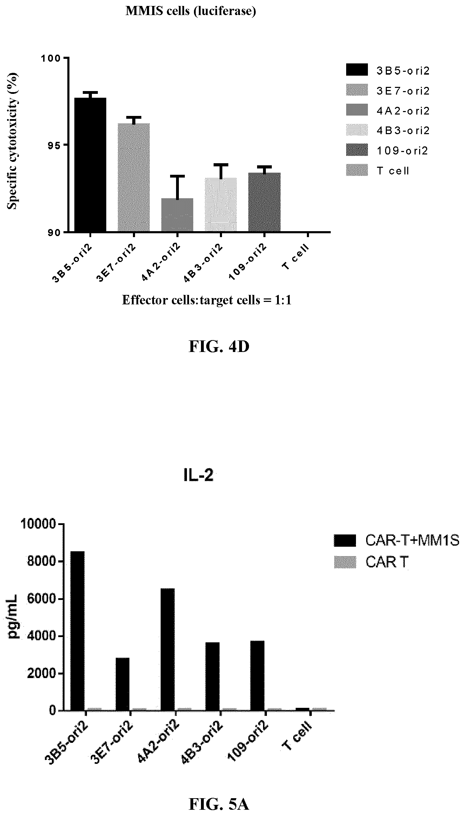

As shown in FIGs. 4A to 4C, compared with blank effector T cells, the results showed that anti-GPRC5D CAR-T cells and the 109 positive control had a gradient-dependent significant killing effect on the target cells, without killing effect on non-target cells.

Another method for detecting the killing ability included co-incubating MM1S-lucifurase as the target cells with the effector CAR-T cells for 6 hours, and determining a killing effect by measuring the luciferase value in the remaining target cells. The effector/target ratio was 1:1, and the detection results were shown in 4D. The results showed that, compared with T cells, the CAR-T cells in the present application had a significant killing effect.

**Example 8. Cytokine Secretion after in vitro killing by GPRC5D CAR-T Cells**

Referring to Example 7, a target cell killing assay was carried out in a 96-well plate, with the ratio of effector cells to target cells as 1:1; then the number of effector cells in each of the 96 well was fixed at 2×10$^4$; the target cells and the effector cells were added in sequence; and 2 repeat wells were set under each effector/target ratio in each group, and 2 separate effector cell wells were additionally arranged for each group to detect the secretion of background factors of the effector cells.

Approximately 24 hours after target cell killing, centrifugation was performed on the 96-well plate to collect the supernatant from each well (in case of no immediate assay, the supernatant needed to be preserved in a -80°C refrigerator).

The effector cells were centrifuged to remove the supernatant, and diluted to 3×10$^5$ cells/mL with X-VIVO, and 100 μL was taken from each well for later use. Target-free cells (i.e., CAR-T cells not co-cultured with tumor cells) and MM1S cells were mixed in a round-bottom 96-well plate at an effector/target ratio of 1:1; and after 24 h of co-incubation, the supernatant was sucked, and the contents of IL-2 and IFN-γ in the supernatant were detected by the R&D Cytokine Kit.

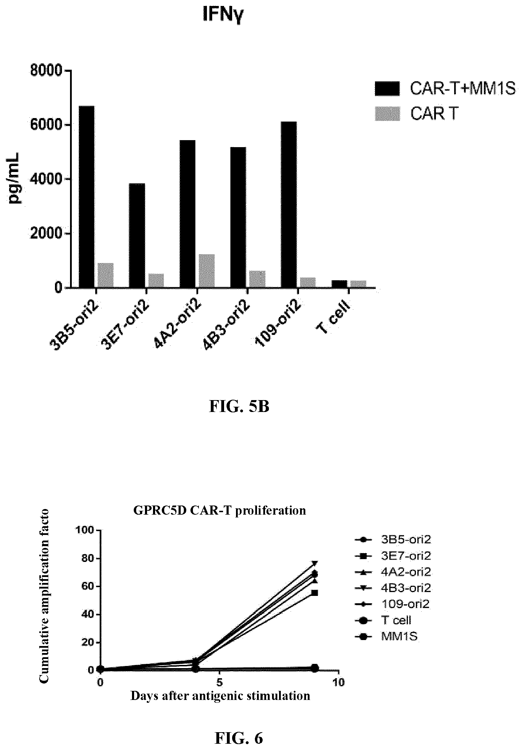

As shown in FIG. 5, without MM1S cell stimulation, CAR-T cells in each group secreted little IL-2 and IFN-γ, and with MM1S cell stimulation, the present application could produce more IFN-γ secretion when killing target cells as compared with the positive control 109-ori2 selected in the present application.

**Example 9. Targeted Proliferation Ability of GPRC5D CAR-T cells**

In this example, the targeted proliferation ability of the CAR-T cells was detected by selecting untreated MM1S cells as target cells.

Generally, the CAR-T cells were cultured on about Days 9-12, before which the CAR positive rate of each group needed to be detected in advance, and then the CAR positive rate of each group is regulated to be the same by using blank effector T cells (the medium was an X-VIVO 15 medium free of any additives).

The CAR-T cells and the MM1S cells were subjected to the first round of targeted stimulation at a effector/target ratio of 1:1; after 4-5 days of co-incubation, all the cells were collected, sampled and counted, and then subjected to the second round of targeted stimulation at the ratio of 1:3 (all CAR-T cells:new MM1S cells); and after 4-5 days of another co-incubation, the third round of targeted stimulation was performed (the ratio between the two types of cells was the same as that in the second round of targeted stimulation). During the three rounds of targeted stimulation, a proper amount of X-VIVO 15 medium was supplemented every 1-2 days according to the cell growth.

As shown in FIG. 6, after two or three rounds of targeted stimulation of the GPRC5D CAR-T cells, the CAR-T proliferation and amplification factor of the present application was comparable to that of the positive control. However, no amplification was substantially observed in the co-incubation wells of T cells and the individual MM1S target cell wells.

### Example 10. In vivo Assay of GPRC5D CAR-T in Mice

We further verified the tumor elimination and inhibition effects of GPRC5D CAR-T cells in NSG mice. Specifically, the assay included three groups, including a T cell group, a positive control group and a GPRC5D CAR-T cell group, with 5 mice in each group. MM1S cells were first inoculated subcutaneously to the NSG mice at a dose of $1\times10^7$ cells/mouse; after about 2 weeks (tumor size of 50-150 mm$^3$), their tail veins were injected with the CAR-T (or T) cells from three groups that had been cultured for 10-12 days, at a dose of $2\times10^6$ CAR-T cells/mouse (the total number of inoculated cells in each mouse was regulated to be the same in terms of T cells). After the CAR-T (or T) cells were inoculated, the secretion of cytokines in the peripheral blood of mice was detected on Days 7 and 14 after inoculation of the CAR-T (or T) cells, and the tumor size and body weight of the mice were measured 3 times a week and observed for 3 weeks.

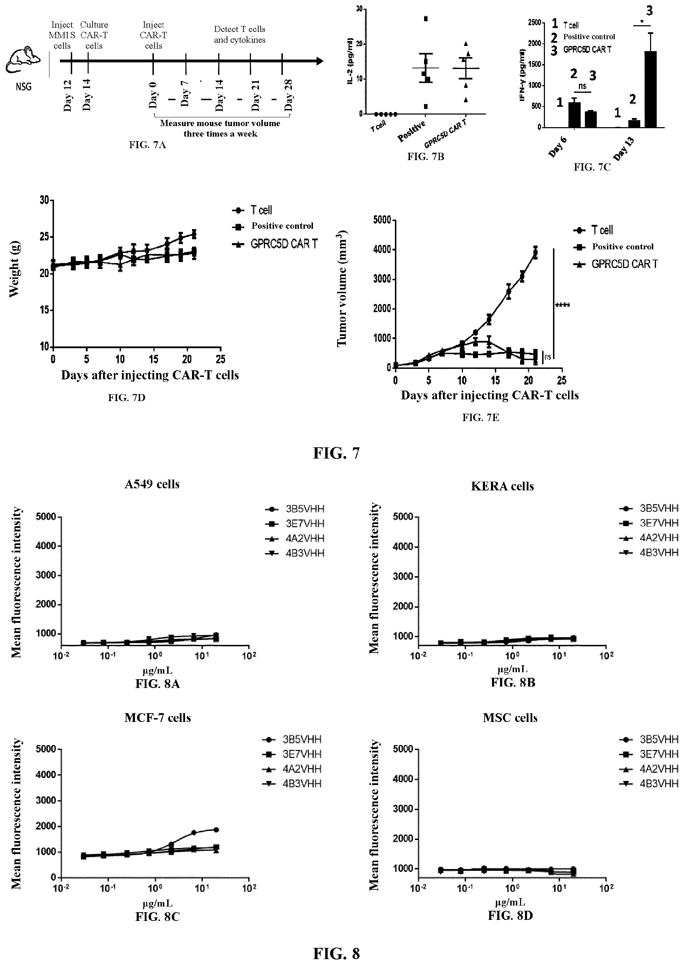

In terms of cytokine secretion, as shown in FIGs. 7B and 7C, the GPRC5D CAR-T cell group could produce a larger amount of IFN-γ in mice and for a longer period (the IFN-γ detected on Day 14 after CAR-T injection was significantly more than that of the positive control group), but the secretion of other cytokines such as IL-2 was very low, with slight difference between groups.

In terms of tumor reduction rate, as shown in FIG. 7E, the GPRC5D CAR-T cells were substantially the same as those of the positive control group, and showed a significant tumor reduction effect compared with the T cell group.

As described in FIG. 7D, after the NSG mice were inoculated with CAR-T (or T) cells, their body weight was weighed every 2-3 days and plotted in curves, and the hair color, excretion, diet and water intake, physical movement, and death of the mice were observed and recorded. The body weight of the mice in the GPRC5D CAR-T cell and positive control groups increased steadily over time, showing the trends consistent with that of the body weight curve of mice in the T group. During the assay, the mice in the GPRC5D CAR-T cell and positive control groups had bright hair color, and normal behaviors, diet and water intake, and urine color, demonstrating good safety of the GPRC5D CAR-T cells in mice.

### Example 11. Specificity Assay for GPRC5D Antibodies

The assay on the non-specific binding of the target antigen (GPRC5D) on the cell surface to the antibodies (i.e., the 3B5, 3E7, 4A2, 4B3, 3E9, 3F5, 3G4, 4A4 and 506H9 antibodies obtained in Example 2) was performed by flow cytometry fluorescence-activated cell sorting (FACS) using an iQue Screener flow cytometer (purchased from IntelliCyt) and PBS containing 0.1% BSA as buffer. Target cells (i.e., cells that do not express GPRC5D antigens, such as A549 cells, KERA cells, MCF-7 cells, and MSC cells) with a concentration of $1\times10^6$ cells/mL were prepared with a buffer, and added to a 96-well pointed-bottom plate (corning 3894), with 30 μL per well. The assay antibodies with the concentration of 20 μg/mL were prepared with a buffer, and diluted at a 3-fold ratio to create 8 concentration gradients; the prepared antibodies at different concentrations were added to the pre-plated target cells at 30 μL/well and mixed well; the resultant was incubated for 1 hour in a refrigerator at 4°C; 150 μL of buffer was added to each well, and incubated for 1 h in a refrigerator at 4°C; 150 μL of buffer was added to each well; centrifugation was performed for 5 min at 300 g; the supernatant was discarded and the cells were loosened by shaking; washing was repeated once. Fluorescent secondary antibodies (ab98593) was prepared using the buffer at a ratio of 1:200, followed by the addition of 30 μl per well to the cells and homogenous mixing, and the resultant was incubated for 30 min in the refrigerator at 4 °C; and 150 μL of buffer was added to each well, centrifugation was performed for 5 min at 300 g, the supernatant was discarded, and the cells were loosened by shaking and washed twice. 35 μL of buffer was added to each well and mixed well, followed by detection with the flow cytometer.

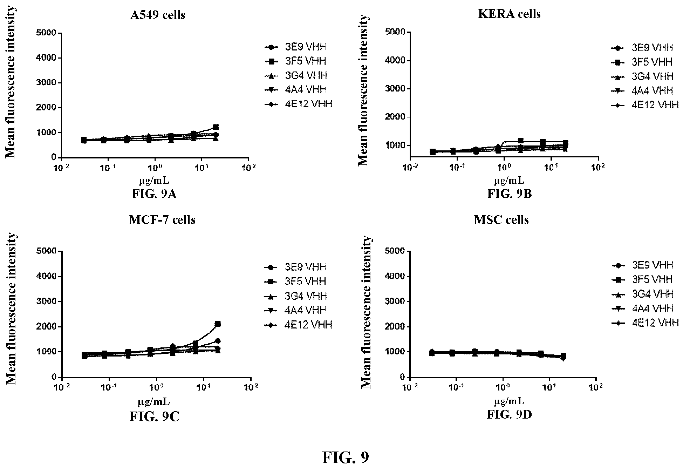

The analysis results of the flow cytometry affinity binding assay were shown in FIGs. 8A-8D and FIGs. 9A-9D, where all the anti-GPRCSD 3B5, 3E7, 4A2, 4B3, 3E9, 3F5, 3G4, 4A4 and 4E12 antibodies do not bind to the above cells non-specifically, indicating that the anti-GPRCSD antibodies had strong specificity.

**Example 12. Endocytosis Assay of GPRC5D Antibodies**

Wild-type cell line MM.1S cells and NCIH929-GPRC5D cells overexpressing GPRC5D target antigens were selected, and the specific operation was as follows: the target cells were plated at $5\times10^4$ cells/well into a 96-well V-bottom plate, with 30 μL per well, and a total of two plates (Plate A and Plate B); the antibodies were diluted at a starting concentration of 20 μg/mL and at a gradient of 3-fold ratio, with a total of 7 concentration gradients; and the antibodies were added at 30 μL/well to the target cell-plated 96-well V bottom plate, mixed well and incubated for 1 h, followed by washing once with PBS containing 0.1% BSA. Reference antibodies were diluted at 1:200; anti-human IgG-Fc (Dylight650) secondary antibodies were added at 30 μL/well; the plates were incubated for 30 min at 4°C, and washed once with PBS containing 0.1% BSA; then 50 μL of complete medium (RPMI1640+10%FBS) was added; the plates A and B were incubated for 2 hours in an incubator at 4°C and 37°C, respectively; the PBS containing 0.1% BSA was added for washing once; 30 μL of fluorescence quenching buffer was added to each well; the plates were kept at 4°C for 10 min; the PBS containing 0.1% BSA was added for washing once; the resultant was resuspended at 30 μL/well with the PBS containing 0.1% BSA, and then detected by the intellicyt iQue3 flow cytometer.

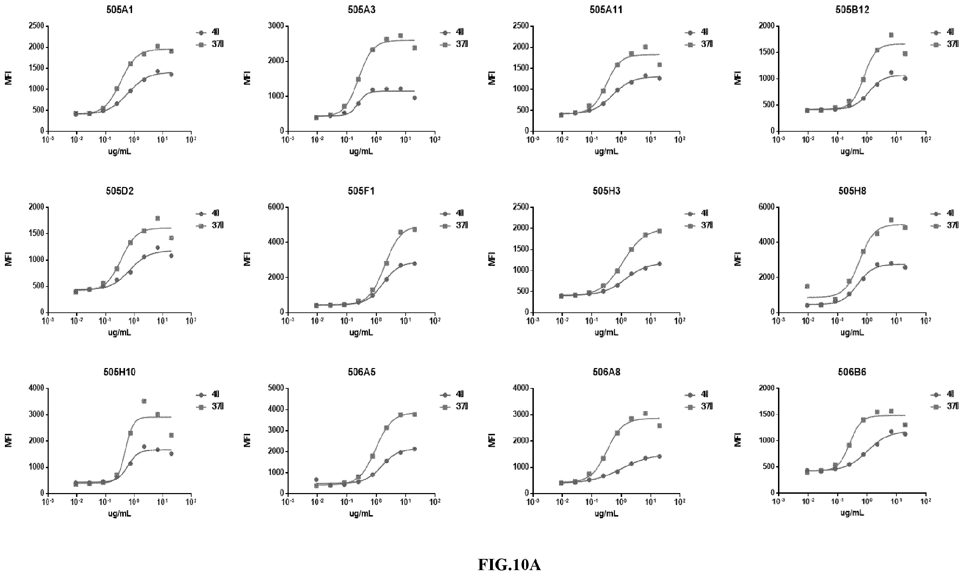

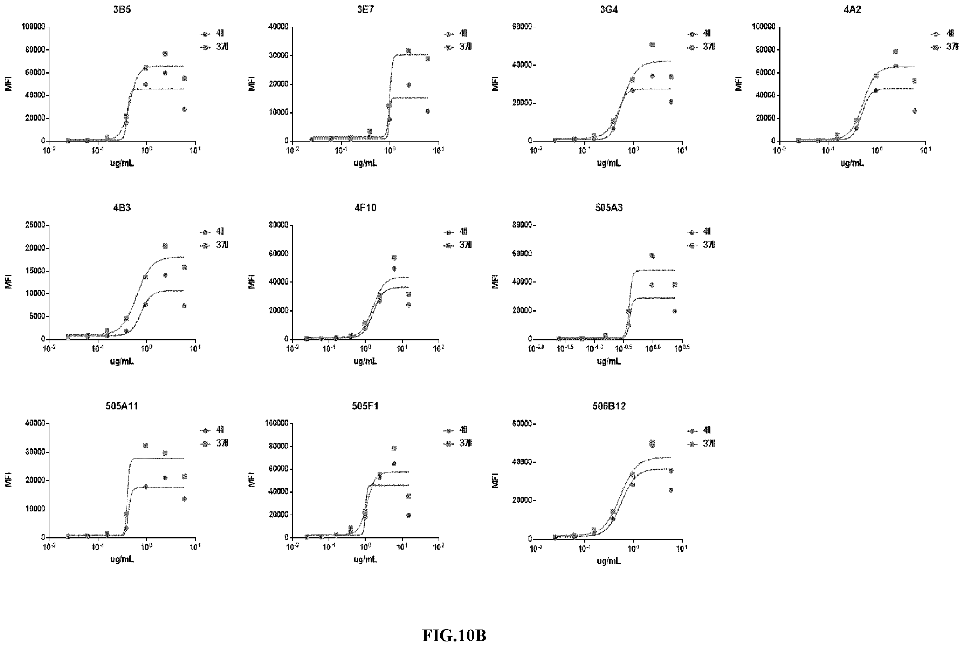

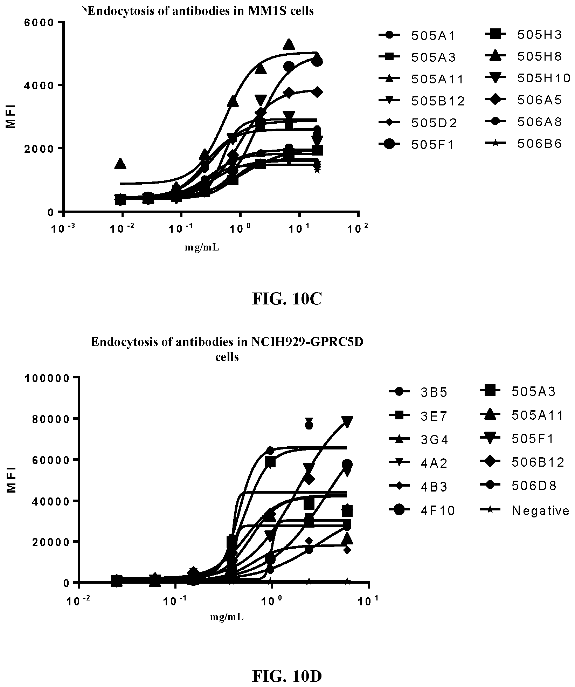

The results were shown in FIGs. 10A-10D, indicating that the GPRC5D antibodies provided by the present application all showed endocytosis to different extents at 37°C.

The foregoing detailed description is provided by way of explanation and examples, and is not intended to limit the scope of the appended claims. Diverse variations of the embodiments listed in the present application until now would be obvious to those of ordinary skills in the art, and should be kept within the scope of the appended claims and equivalents thereof.

**Claims**

1.   An isolated antigen binding protein capable of binding to a G protein-coupled receptor, class C, group 5, member D GPRC5D protein with an $EC_{50}$ value below 1.0 μg/mL.

2.   The antigen binding protein according to claim 1, comprising an antibody or an antigen-binding fragment thereof.

3.   The antigen binding protein according to any one of claims 1-2, wherein said antigen-binding fragment comprises Fab, Fab', F(ab)$_2$, an Fv fragment, F(ab')$_2$, scFv, di-scFv, VHH and/or dAb.

4.   The antigen binding protein according to any one of claims 2-3, wherein said antigen-binding fragment is VHH.

5.   The antigen binding protein according to any one of claims 2-4, wherein said antibody is selected from the group consisting of: a monoclonal antibody, a chimeric antibody, a humanized antibody, and a fully human antibody.

6.   The antigen binding protein according to any one of claims 1-5, which is capable of competing with a reference antibody for binding to said GPRC5D protein, wherein said reference antibody comprises an antibody heavy-chain variable region VH comprising HCDR1, HCDR2, and HCDR3, and said reference antibody comprises any set of amino acid sequences selected from the group consisting of:

 (1) HCDR1: SEQ ID NO: 102, HCDR2: SEQ ID NO: 103, and HCDR3: SEQ ID NO: 104;
 (2) HCDR1: SEQ ID NO: 105, HCDR2: SEQ ID NO: 106, and HCDR3: SEQ ID NO: 107;
 (3) HCDR1: SEQ ID NO: 109, HCDR2: SEQ ID NO: 110, and HCDR3: SEQ ID NO: 111;
 (4) HCDR1: SEQ ID NO: 105, HCDR2: SEQ ID NO: 114, and HCDR3: SEQ ID NO: 115;
 (5) HCDR1: SEQ ID NO: 116, HCDR2: SEQ ID NO: 117, and HCDR3: SEQ ID NO: 118;
 (6) HCDR1: SEQ ID NO: 119, HCDR2: SEQ ID NO: 120, and HCDR3: SEQ ID NO: 121;
 (7) HCDR1: SEQ ID NO: 123, HCDR2: SEQ ID NO: 124, and HCDR3: SEQ ID NO: 125;
 (8) HCDR1: SEQ ID NO: 127, HCDR2: SEQ ID NO: 128, and HCDR3: SEQ ID NO: 129;
 (9) HCDR1: SEQ ID NO: 130, HCDR2: SEQ ID NO: 131, and HCDR3: SEQ ID NO: 132;
 (10) HCDR1: SEQ ID NO: 95, HCDR2: SEQ ID NO: 96, and HCDR3: SEQ ID NO: 97;
 (11) HCDR1: SEQ ID NO: 133, HCDR2: SEQ ID NO: 134, and HCDR3: SEQ ID NO: 135;
 (12) HCDR1: SEQ ID NO: 136, HCDR2: SEQ ID NO: 134, and HCDR3: SEQ ID NO: 137;
 (13) HCDR1: SEQ ID NO: 138, HCDR2: SEQ ID NO: 139, and HCDR3: SEQ ID NO: 140;
 (14) HCDR1: SEQ ID NO: 142, HCDR2: SEQ ID NO: 143, and HCDR3: SEQ ID NO: 144;

(15) HCDR1: SEQ ID NO: 145, HCDR2: SEQ ID NO: 146, and HCDR3: SEQ ID NO: 147;
(16) HCDR1: SEQ ID NO: 148, HCDR2: SEQ ID NO: 149, and HCDR3: SEQ ID NO: 150;
(17) HCDR1: SEQ ID NO: 152, HCDR2: SEQ ID NO: 153, and HCDR3: SEQ ID NO: 154;
(18) HCDR1: SEQ ID NO: 123, HCDR2: SEQ ID NO: 155, and HCDR3: SEQ ID NO: 156;
(19) HCDR1: SEQ ID NO: 157, HCDR2: SEQ ID NO: 153, and HCDR3: SEQ ID NO: 158;
(20) HCDR1: SEQ ID NO: 159, HCDR2: SEQ ID NO: 160, and HCDR3: SEQ ID NO: 161;
(21) HCDR1: SEQ ID NO: 159, HCDR2: SEQ ID NO: 162, and HCDR3: SEQ ID NO: 163;
(22) HCDR1: SEQ ID NO: 159, HCDR2: SEQ ID NO: 164, and HCDR3: SEQ ID NO: 165;
(23) HCDR1: SEQ ID NO: 166, HCDR2: SEQ ID NO: 167, and HCDR3: SEQ ID NO: 168;
(24) HCDR1: SEQ ID NO: 169, HCDR2: SEQ ID NO: 153, and HCDR3: SEQ ID NO: 170;
(25) HCDR1: SEQ ID NO: 157, HCDR2: SEQ ID NO: 171, and HCDR3: SEQ ID NO: 172;
(26) HCDR1: SEQ ID NO: 174, HCDR2: SEQ ID NO: 175, and HCDR3: SEQ ID NO: 176;
(27) HCDR1: SEQ ID NO: 177, HCDR2: SEQ ID NO: 178, and HCDR3: SEQ ID NO: 179;
(28) HCDR1: SEQ ID NO: 180, HCDR2: SEQ ID NO: 181, and HCDR3: SEQ ID NO: 182;
(29) HCDR1: SEQ ID NO: 184, HCDR2: SEQ ID NO: 106, and HCDR3: SEQ ID NO: 185;
(30) HCDR1: SEQ ID NO: 186, HCDR2: SEQ ID NO: 187, and HCDR3: SEQ ID NO: 188;
(31) HCDR1: SEQ ID NO: 189, HCDR2: SEQ ID NO: 190, and HCDR3: SEQ ID NO: 191;
(32) HCDR1: SEQ ID NO: 159, HCDR2: SEQ ID NO: 192, and HCDR3: SEQ ID NO: 179;
(33) HCDR1: SEQ ID NO: 193, HCDR2: SEQ ID NO: 194, and HCDR3: SEQ ID NO: 195; and
(34) HCDR1: SEQ ID NO: 196, HCDR2: SEQ ID NO: 197, and HCDR3: SEQ ID NO: 198.

7. The antigen binding protein according to any one of claims 1-6, comprising at least one CDR derived from said antibody heavy-chain variable region VH, said VH comprising an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

8. The antigen binding protein according to any one of claims 1-7, comprising HCDR3, said HCDR3 comprising an amino acid sequence as set forth in SEQ ID NO: 215 or SEQ ID NO: 218.

9. The antigen binding protein according to any one of claims 1-8, comprising HCDR3, said HCDR3 comprising an amino acid sequence as set forth in any one of SEQ ID NO: 97, SEQ ID NO: 104, SEQ ID NO: 107, SEQ ID NO: 111, SEQ ID NO: 115, SEQ ID NO: 118, SEQ ID NO: 121, SEQ ID NO: 125, SEQ ID NO: 129, SEQ ID NO: 132, SEQ ID NO: 135, SEQ ID NO: 137, SEQ ID NO: 140, SEQ ID NO: 144, SEQ ID NO: 147, SEQ ID NO: 150, SEQ ID NO: 154, SEQ ID NO: 156, SEQ ID NO: 158, SEQ ID NO: 161, SEQ ID NO: 163, SEQ ID NO: 165, SEQ ID NO: 168, SEQ ID NO: 170, SEQ ID NO: 172, SEQ ID NO: 176, SEQ ID NO: 179, SEQ ID NO: 182, SEQ ID NO: 185, SEQ ID NO: 188, SEQ ID NO: 191, SEQ ID NO: 195 and SEQ ID NO: 198.

10. The antigen binding protein according to any one of claims 1-9, comprising HCDR2, said HCDR2 comprising an amino acid sequence as set forth in $X_1X_2X_3X_4X_5X_6X_7T$, wherein $X_1$ is I or T, $X_2$ is N, SorT, $X_3$ is A, P, R, S or W, $X_4$ is G, R, S, T or none, $X_5$ is D or G, $X_6$ is G or S, and $X_7$ is D, G, I, N, R, S, T or V

11. The antigen binding protein according to any one of claims 1-10, comprising HCDR2, said HCDR2 comprising an amino acid sequence as set forth in any one of SEQ ID NO: 96, SEQ ID NO: 103, SEQ ID NO: 106, SEQ ID NO: 110, SEQ ID NO: 114, SEQ ID NO: 117, SEQ ID NO: 120, SEQ ID NO: 124, SEQ ID NO: 128, SEQ ID NO: 131, SEQ ID NO: 134, SEQ ID NO: 139, SEQ ID NO: 143, SEQ ID NO: 146, SEQ ID NO: 149, SEQ ID NO: 153, SEQ ID NO: 155, SEQ ID NO: 160, SEQ ID NO: 162, SEQ ID NO: 164, SEQ ID NO: 167, SEQ ID NO: 171, SEQ ID NO: 175, SEQ ID NO: 178, SEQ ID NO: 181, SEQ ID NO: 187, SEQ ID NO: 190, SEQ ID NO: 192, SEQ ID NO: 194 and SEQ ID NO: 197.

12. The antigen binding protein according to any one of claims 1-11, comprising HCDR1, said HCDR1 comprising an amino acid sequence as set forth in $GX_2X_3X_4SX_6X_7X_8$, wherein $X_2$ is F, G, I, L, N, R, S or Y, $X_3$ is I or T, $X_4$ is F or L, $X_6$ is I, L, N, R, S, T or Y, $X_7$ is D, N or Y, and $X_8$ is A, D, G, I, N, R, S, T, V or Y

13. The antigen binding protein according to any one of claims 1-12, comprising HCDR1, said HCDR1 comprising an amino acid sequence as set forth in any one of SEQ ID NO: 95, SEQ ID NO: 102, SEQ ID NO: 105, SEQ ID NO:

109, SEQ ID NO: 116, SEQ ID NO: 119, SEQ ID NO: 123, SEQ ID NO: 127, SEQ ID NO: 130, SEQ ID NO: 133, SEQ ID NO: 136, SEQ ID NO: 138, SEQ ID NO: 142, SEQ ID NO: 145, SEQ ID NO: 148, SEQ ID NO: 152, SEQ ID NO: 157, SEQ ID NO: 159, SEQ ID NO: 166, SEQ ID NO: 169, SEQ ID NO: 174, SEQ ID NO: 177, SEQ ID NO: 180, SEQ ID NO: 184, SEQ ID NO: 186, SEQ ID NO: 189, SEQ ID NO: 193 and SEQ ID NO: 196.

14. The antigen binding protein according to any one of claims 1-13, comprising HCDR1, HCDR2, and HCDR3, said HCDR1, HCDR2, and HCDR3 comprising any set of amino acid sequences selected from the group consisting of:

(1) HCDR1: SEQ ID NO: 102, HCDR2: SEQ ID NO: 103, and HCDR3: SEQ ID NO: 104;
(2) HCDR1: SEQ ID NO: 105, HCDR2: SEQ ID NO: 106, and HCDR3: SEQ ID NO: 107;
(3) HCDR1: SEQ ID NO: 109, HCDR2: SEQ ID NO: 110, and HCDR3: SEQ ID NO: 111;
(4) HCDR1: SEQ ID NO: 105, HCDR2: SEQ ID NO: 114, and HCDR3: SEQ ID NO: 115;
(5) HCDR1: SEQ ID NO: 116, HCDR2: SEQ ID NO: 117, and HCDR3: SEQ ID NO: 118;
(6) HCDR1: SEQ ID NO: 119, HCDR2: SEQ ID NO: 120, and HCDR3: SEQ ID NO: 121;
(7) HCDR1: SEQ ID NO: 123, HCDR2: SEQ ID NO: 124, and HCDR3: SEQ ID NO: 125;
(8) HCDR1: SEQ ID NO: 127, HCDR2: SEQ ID NO: 128, and HCDR3: SEQ ID NO: 129;
(9) HCDR1: SEQ ID NO: 130, HCDR2: SEQ ID NO: 131, and HCDR3: SEQ ID NO: 132;
(10) HCDR1: SEQ ID NO: 95, HCDR2: SEQ ID NO: 96, and HCDR3: SEQ ID NO: 97;
(11) HCDR1: SEQ ID NO: 133, HCDR2: SEQ ID NO: 134, and HCDR3: SEQ ID NO: 135;
(12) HCDR1: SEQ ID NO: 136, HCDR2: SEQ ID NO: 134, and HCDR3: SEQ ID NO: 137;
(13) HCDR1: SEQ ID NO: 138, HCDR2: SEQ ID NO: 139, and HCDR3: SEQ ID NO: 140;
(14) HCDR1: SEQ ID NO: 142, HCDR2: SEQ ID NO: 143, and HCDR3: SEQ ID NO: 144;
(15) HCDR1: SEQ ID NO: 145, HCDR2: SEQ ID NO: 146, and HCDR3: SEQ ID NO: 147;
(16) HCDR1: SEQ ID NO: 148, HCDR2: SEQ ID NO: 149, and HCDR3: SEQ ID NO: 150;
(17) HCDR1: SEQ ID NO: 152, HCDR2: SEQ ID NO: 153, and HCDR3: SEQ ID NO: 154;
(18) HCDR1: SEQ ID NO: 123, HCDR2: SEQ ID NO: 155, and HCDR3: SEQ ID NO: 156;
(19) HCDR1: SEQ ID NO: 157, HCDR2: SEQ ID NO: 153, and HCDR3: SEQ ID NO: 158;
(20) HCDR1: SEQ ID NO: 159, HCDR2: SEQ ID NO: 160, and HCDR3: SEQ ID NO: 161;
(21) HCDR1: SEQ ID NO: 159, HCDR2: SEQ ID NO: 162, and HCDR3: SEQ ID NO: 163;
(22) HCDR1: SEQ ID NO: 159, HCDR2: SEQ ID NO: 164, and HCDR3: SEQ ID NO: 165;
(23) HCDR1: SEQ ID NO: 166, HCDR2: SEQ ID NO: 167, and HCDR3: SEQ ID NO: 168;
(24) HCDR1: SEQ ID NO: 169, HCDR2: SEQ ID NO: 153, and HCDR3: SEQ ID NO: 170;
(25) HCDR1: SEQ ID NO: 157, HCDR2: SEQ ID NO: 171, and HCDR3: SEQ ID NO: 172;
(26) HCDR1: SEQ ID NO: 174, HCDR2: SEQ ID NO: 175, and HCDR3: SEQ ID NO: 176;
(27) HCDR1: SEQ ID NO: 177, HCDR2: SEQ ID NO: 178, and HCDR3: SEQ ID NO: 179;
(28) HCDR1: SEQ ID NO: 180, HCDR2: SEQ ID NO: 181, and HCDR3: SEQ ID NO: 182;
(29) HCDR1: SEQ ID NO: 184, HCDR2: SEQ ID NO: 106, and HCDR3: SEQ ID NO: 185;
(30) HCDR1: SEQ ID NO: 186, HCDR2: SEQ ID NO: 187, and HCDR3: SEQ ID NO: 188;
(31) HCDR1: SEQ ID NO: 189, HCDR2: SEQ ID NO: 190, and HCDR3: SEQ ID NO: 191;
(32) HCDR1: SEQ ID NO: 159, HCDR2: SEQ ID NO: 192, and HCDR3: SEQ ID NO: 179;
(33) HCDR1: SEQ ID NO: 193, HCDR2: SEQ ID NO: 194, and HCDR3: SEQ ID NO: 195; and
(34) HCDR1: SEQ ID NO: 196, HCDR2: SEQ ID NO: 197, and HCDR3: SEQ ID NO: 198.

15. The antigen binding protein according to any one of claims 1-14, comprising an antibody heavy-chain variable region VH, wherein said VH comprises a framework region H-FR1, a C-terminal of said H-FR1 is directly or indirectly linked to an N-terminal of said HCDR1, and said H-FR1 comprises an amino acid sequence as set forth in SEQ ID NO: 98.

16. The antigen binding protein according to claim 15, wherein said VH comprises a framework region H-FR2 located between said HCDR1 and said HCDR2, and said H-FR2 comprises an amino acid sequence as set forth in SEQ ID NO: 216.

17. The antigen binding protein according to claim 16, wherein said H-FR2 comprises an amino acid sequence as set forth in any one of SEQ ID NO: 99, SEQ ID NO: 108, SEQ ID NO: 112, SEQ ID NO: 122, SEQ ID NO: 151, and SEQ ID NO: 173.

18. The antigen binding protein according to any one of claims 15-17, wherein said VH comprises a framework region H-FR3 located between said HCDR2 and said HCDR3, and said H-FR3 comprises an amino acid sequence as set forth in SEQ ID NO: 217.

19. The antigen binding protein according to claims 18, wherein said H-FR3 comprises an amino acid sequence as set forth in any one of SEQ ID NO: 100, SEQ ID NO: 113, SEQ ID NO: 126, SEQ ID NO: 141, and SEQ ID NO: 183.

20. The antigen binding protein according to any one of claims 15-19, wherein said VH comprises a framework region H-FR4, an N-terminal of said H-FR4 is linked to a C-terminal of said HCDR3, and said H-FR4 comprises an amino acid sequence as set forth in SEQ ID NO: 101.

21. The antigen binding protein according to any one of claims 1-20, comprising an antibody heavy-chain variable region VH, said VH comprising an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

22. The antigen binding protein according to any one of claims 1-21, which is VHH, said VHH comprising an amino acid sequence as set forth in any one of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

23. The antigen binding protein according to any one of claims 1-22, comprising an antibody heavy-chain constant region derived from IgG.

24. The antigen binding protein according to any one of claims 1-23, comprising an antibody heavy-chain constant region derived from human IgG.

25. The antigen binding protein according to any one of claims 1-24, comprising an antibody heavy-chain constant region derived from human IgG1.

26. A chimeric antigen receptor comprising a targeting moiety, said targeting moiety comprising the antigen binding protein of any one of claims 1-25.

27. The chimeric antigen receptor according to claim 26, comprising a co-stimulatory signal region, wherein said co-stimulatory signal region comprises an intracellular co-stimulatory signal region derived from one or more proteins selected from the group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD83 ligands, CD40, and MyD88.

28. The chimeric antigen receptor according to claim 27, wherein said co-stimulatory signal region is a 4-1BB-derived intracellular co-stimulatory signal region.

29. The chimeric antigen receptor according to any one of claims 27-28, wherein said co-stimulatory signal region comprises an amino acid sequence as set forth in SEQ ID NO: 78.

30. The chimeric antigen receptor according to any one of claims 27-29, comprising an intracellular signal region, said intracellular signal region comprising an intracellular signal region derived from one or more proteins selected from the group consisting of: CD3ζ, CD3δ, CD3γ, CD3ε, CD79a, CD79b, FcεRIγ, FcεRIβ, FcγRIIa, bovine leukemia virus gp30, Epstein-Barr virus EBV LMP2A, simian immunodeficiency virus PBj 14 Nef, Kaposi sarcoma-associated herpesvirus HSKV, DAP10, DAP-12 and a domain comprising at least one ITAM domain.

31. The chimeric antigen receptor according to claim 30, wherein said intracellular signal region is a CD3ζ-derived signaling domain.

32. The chimeric antigen receptor according to claim 31, wherein said intracellular signal region comprises an amino acid sequence as set forth in SEQ ID NO: 80.

33. The chimeric antigen receptor according to any one of claims 26-32, comprising a transmembrane region, said transmembrane region comprising a transmembrane region derived from one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154 and SLAM.

34. The chimeric antigen receptor according to claim 33, wherein said transmembrane region is a CD8-derived transmembrane region.

35. The chimeric antigen receptor according to claim 34, wherein said transmembrane region comprises an amino acid sequence as set forth in SEQ ID NO: 76.

36. The chimeric antigen receptor according to any one of claims 34-35, comprising a hinge region between said targeting moiety and said transmembrane region, said hinge region comprising a hinge region derived from one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30 and LIGHT.

37. The chimeric antigen receptor according to claim 36, wherein said hinge region is a CD8-derived hinge region.

38. The chimeric antigen receptor according to claim 37, wherein said hinge region comprises an amino acid sequence as set forth in SEQ ID NO: 74.

39. The chimeric antigen receptor according to any one of claims 26-38, further comprising a low-density lipoprotein receptor-related protein or a fragment thereof, said low-density lipoprotein receptor-related protein or the fragment thereof being located at said C-terminal of said intracellular signal region.

40. The chimeric antigen receptor according to claim 39, wherein said low-density lipoprotein receptor-related protein or the fragment thereof comprises one or more selected from the group consisting of: low-density lipoprotein receptor-related proteins 1-12 or fragments thereof.

41. The chimeric antigen receptor according to any one of claims 39-40, wherein said low-density lipoprotein receptor-related protein or the fragment thereof is a low-density lipoprotein receptor-related protein 6 or a fragment thereof.

42. The chimeric antigen receptor according to any one of claims 39-41, wherein said low-density lipoprotein receptor-related protein or the fragment thereof comprises an amino acid sequence as set forth in SEQ ID NO: 84.

43. The chimeric antigen receptor according to any one of claims 26-42, further comprising a signal peptide.

44. The chimeric antigen receptor according to claim 43, wherein said signal peptide is a CD8 protein-derived signal peptide.

45. The chimeric antigen receptor according to claim 44, wherein said signal peptide comprises an amino acid sequence as set forth in SEQ ID NO: 72.

46. The chimeric antigen receptor according to any one of claims 26-45, comprising an amino acid sequence as set forth in any one of SEQ ID NO: 86, SEQ ID NO: 88, SEQ ID NO: 90, SEQ ID NO: 92, SEQ ID NO: 200, SEQ ID NO: 201, SEQ ID NO: 202, SEQ ID NO: 203 and SEQ ID NO: 204.

47. The chimeric antigen receptor according to any one of claims 26-46, comprising a nucleotide sequence as set forth in any one of SEQ ID NO: 85, SEQ ID NO: 87, SEQ ID NO: 89, and SEQ ID NO: 91.

48. A polypeptide, comprising the antigen binding protein of any one of claims 1-25.

49. An isolated nucleic acid molecule or isolated nucleic acid molecules, encoding the antigen binding protein of any one of claims 1-25 and/or the chimeric antigen receptor of any one of claims 26-47.

50. The nucleic acid molecule according to claim 49, further comprising a promoter.

**51.** The nucleic acid molecule according to claim 50, wherein said promoter is a constitutive promoter.

**52.** The nucleic acid molecule according to any one of claims 50-51, wherein said promoter is an EF1α promoter.

**53.** The nucleic acid molecule according to any one of claims 49-52, comprising a nucleotide sequence as set forth in any one of SEQ ID NO: 85, SEQ ID NO: 87, SEQ ID NO: 89, and SEQ ID NO: 91.

**54.** A vector, comprising the nucleic acid molecule of any one of claims 49-53.

**55.** The vector according to claim 54, which is a viral vector.

**56.** The vector according to any one of claims 54-55, which is a lentiviral vector.

**57.** A cell, comprising the antigen binding protein of any one of claims 1-25, the chimeric antigen receptor of any one of claims 26-47, the nucleic acid molecule of any one of claims 49-53, and/or the vector of any one of claims 54-56.

**58.** The cell according to claim 57, which is an immune effector cell.

**59.** The cell according to any one of claims 57-58, comprising a T cell, a B cell, a natural killer NK cell, a macrophage, a NKT cell, a monocyte, a dendritic cell, a granulocyte, a lymphocyte, a leukocyte, a peripheral blood mononuclear cell, an embryonic stem cell, a lymphoprogenitor cell, and/or a pluripotent stem cell.

**60.** The cell according to any one of claims 57-59, which is a T cell.

**61.** A method for preparing the antigen binding protein of any one of claims 1-25 and/or the chimeric antigen receptor of any one of claims 26-47, comprising culturing the cell of any one of claims 57-60 under a condition enabling expression of the antigen binding protein of any one of claims 1-25 and/or the chimeric antigen receptor of any one of claims 26-47.

**62.** A method for preparing a modified immune effector cell, comprising introducing the vector of any one of claims 55-56 into an immune effector cell.

**63.** A pharmaceutical composition, comprising the antigen binding protein of any one of claims 1-25, the chimeric antigen receptor of any one of claims 26-47, the polypeptide of claim 48, the nucleic acid molecule of any one of claims 49-53, the vector of any one of claims 54-56, and/or the cell of any one of claims 57-60, and optionally, a pharmaceutically acceptable carrier.

**64.** Use of the antigen binding protein of any one of claims 1-25, the chimeric antigen receptor of any one of claims 26-47, the polypeptide of claim 48, the nucleic acid molecule of any one of claims 49-53, the vector of any one of claims 54-56, the cell of any one of claims 57-60, and/or the pharmaceutical composition of claim 63 in preparation of a medicament for treating, preventing and/or relieving diseases or disorders associated with abnormal expression of GPRC5D.

**65.** The use according to claim 64, wherein said diseases or disorders associated with abnormal expression of GPRC5D comprise tumors.

**66.** The use according to claim 65, wherein said tumors comprise solid tumors.

**67.** The use according to claim 65, wherein said tumors comprise non-solid tumors.

**68.** The use according to claim 65, wherein said tumors comprise hematologic neoplasms and/or lymphomas.

**69.** The use according to claim 65, wherein said tumors comprise myelomas.

**70.** A method for preventing, treating and/or relieving diseases or disorders associated with abnormal expression of GPRC5D, comprising administering the antigen binding protein of any one of claims 1-25, the chimeric antigen receptor of any one of claims 26-47, the polypeptide of claim 48, the nucleic acid molecule of any one of claims 49-53, the vector of any one of claims 54-56, the cell of any one of claims 57-60, and/or the pharmaceutical composition of claim 63 to a subject in need thereof.

71. The method according to claim 70, wherein said diseases or disorders associated with abnormal expression of GPRC5D comprise tumors.

72. The method according to claim 71, wherein said tumors comprise solid tumors.

73. The method according to claim 71, wherein said tumors comprise non-solid tumors.

74. The method according to claim 71, wherein said tumors comprise hematologic neoplasms and/or lymphomas.

75. The method according to claim 71, wherein said tumors comprise myelomas.

**CHO-GPRC5D cells**

FIG. 1A

**CHO-GPRC5D cells**

FIG. 1B

**CHO-GPRC5D cells**

FIG. 1C

**K562-GPRC5D cells**

FIG. 1D

**K562-GPRC5D cells**

FIG. 1E

**K562-GPRC5D cells**

FIG. 1F

**MM1S cells**

**FIG. 1G**

**MM1S cells**

**FIG. 1H**

**MM1S cells**

**FIG. 1I**

FIG. 1J

FIG. 2

**GPRC5D CAR-T proliferation**

FIG. 3A

**GPRC5D CAR-T proliferation**

FIG. 3B

FIG. 3C

**CHO-GPRC5D cells**

**FIG. 4A**

GPRC5D CAR-T cell killing toxicity

**FIG. 4B**

**GPRC5D CAR-T cell killing toxicity**

**FIG. 4C**

MMIS cells (luciferase)

FIG. 4D

IL-2

FIG. 5A

## IFNγ

**FIG. 5B**

**FIG. 6**

FIG. 7A

FIG. 7B

FIG. 7C

FIG. 7D

FIG. 7E

**FIG. 7**

A549 cells

FIG. 8A

KERA cells

FIG. 8B

MCF-7 cells

FIG. 8C

MSC cells

FIG. 8D

**FIG. 8**

41

FIG. 9A — A549 cells

FIG. 9B — KERA cells

FIG. 9C — MCF-7 cells

FIG. 9D — MSC cells

FIG. 9

FIG.10A

**FIG.10B**

**FIG. 10C**

**FIG. 10D**

## INTERNATIONAL SEARCH REPORT

| International application No. |
|---|
| **PCT/CN2022/115590** |

**A. CLASSIFICATION OF SUBJECT MATTER**

C07K 16/28(2006.01)i; C07K 16/30(2006.01)i; A61P 35/00(2006.01)i; A61P 39/00(2006.01)i

According to International Patent Classification (IPC) or to both national classification and IPC

**B. FIELDS SEARCHED**

Minimum documentation searched (classification system followed by classification symbols)

C07K 16/-; A61P 35/-; A61P 39/-

Documentation searched other than minimum documentation to the extent that such documents are included in the fields searched

Electronic data base consulted during the international search (name of data base and, where practicable, search terms used)

CNABS; CNTXT; VEN; DWPI; CJFD; STN; CNKI; 百度, BAIDU; 中国专利生物序列检索, Chinese Patent Biological Sequence Retrieval; PUBMED; Genbank: GPRC5D, 抗体, 抗原结合蛋白, 嵌合抗原受体, 骨髓瘤, EC50, SEQ ID NO: 4-70; SEQ ID NO: 96-198; antibody; antigen binding protein; CAR-T

**C. DOCUMENTS CONSIDERED TO BE RELEVANT**

| Category* | Citation of document, with indication, where appropriate, of the relevant passages | Relevant to claim No. |
|---|---|---|
| X | WO 2020092854 A2 (JUNO THERAPEUTICS INC. et al.) 07 May 2020 (2020-05-07) see claims 1, 47-48, 50-52, 54, 56-58, 68, 118-123, 145, 180, 183-185, and 222-226 | 1-75 |
| X | CN 110462038 A (DAIICHI SANKYO COMPANY, LIMITED) 15 November 2019 (2019-11-15) see abstract, and claims 1-41 | 1-25, 48-75 |
| X | CN 111788231 A (F. HOFFMANN-LA ROCHE AG.) 16 October 2020 (2020-10-16) see abstract, and claims 1-7 and 28-41 | 1-25, 48-75 |
| A | COHEN, Y. et al. ""GPRC5D is a promising marker for monitoring the tumor load and to target multiple myeloma cells"" *Hematology,* Vol. 18, No. 6, 15 November 2013 (2013-11-15), pp. 348-351 | 1-75 |
| A | KODAMA, T. et al. ""Anti-GPRC5D/CD3 bispecific T-cell-redirecting antibody for the treatment of multiple myeloma"" *Molecular cancer therapeutics,* Vol. 18, No. 9, 03 July 2019 (2019-07-03), pp. 1555-1564 | 1-75 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **11 November 2022** | **25 November 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/115590** |

**C. DOCUMENTS CONSIDERED TO BE RELEVANT**

| Category* | Citation of document, with indication, where appropriate, of the relevant passages | Relevant to claim No. |
|---|---|---|
| A | DE LARREA, C. F. et al. ""Defining an optimal dual-targeted CAR T-cell therapy approach simultaneously targeting BCMA and GPRC5D to prevent BCMA escape-driven relapse in multiple myeloma"" *Blood Cancer Discov*, Vol. 1, No. 2, 01 September 2020 (2020-09-01), pp. 146-154 | 1-75 |

Form PCT/ISA/210 (second sheet) (January 2015)

**INTERNATIONAL SEARCH REPORT**

International application No.

**PCT/CN2022/115590**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

   ☑ in the form of an Annex C/ST.25 text file.

   ☐ on paper or in the form of an image file.

   b. ☐ furnished together with the international application under PCT Rule 13ter.1(a) for the purposes of international search only in the form of an Annex C/ST.25 text file.

   c. ☐ furnished subsequent to the international filing date for the purposes of international search only:

   ☐ in the form of an Annex C/ST.25 text file (Rule 13ter.1(a)).

   ☐ on paper or in the form of an image file (Rule 13ter.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   [1] The actually submitted sequence table is an XML file in Standard ST.26.

Form PCT/ISA/210 (continuation of first sheet) (January 2015)

## INTERNATIONAL SEARCH REPORT

| International application No. |
| --- |
| **PCT/CN2022/115590** |

| Box No. II     Observations where certain claims were found unsearchable (Continuation of item 2 of first sheet) |
| --- |

This international search report has not been established in respect of certain claims under Article 17(2)(a) for the following reasons:

1. ☑ Claims Nos.: **70-75**
   because they relate to subject matter not required to be searched by this Authority, namely:

   [1] Claims 70-75 relate to a method for preventing, treating and/or alleviating diseases or disorders associated with the abnormal expression of GPRC5D, belonging to a disease treatment method defined in PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

# EP 4 397 686 A1

**INTERNATIONAL SEARCH REPORT**
Information on patent family members

International application No.

**PCT/CN2022/115590**

| Patent document cited in search report | | | Publication date (day/month/year) | Patent family member(s) | | | Publication date (day/month/year) |
|---|---|---|---|---|---|---|---|
| WO | 2020092854 | A2 | 07 May 2020 | CL | 2021001153 | A1 | 07 January 2022 |
| | | | | TW | 202021981 | A | 16 June 2020 |
| | | | | CA | 3116413 | A1 | 07 May 2020 |
| | | | | IL | 282666 | A | 30 June 2021 |
| | | | | JP | 2022506598 | A | 17 January 2022 |
| | | | | CO | 2021007254 | A2 | 30 August 2021 |
| | | | | CN | 113614108 | A | 05 November 2021 |
| | | | | EP | 3873937 | A2 | 08 September 2021 |
| | | | | US | 2021393689 | A1 | 23 December 2021 |
| | | | | SG | 11202103873V | A | 28 May 2021 |
| | | | | PE | 20211058 | A1 | 07 June 2021 |
| | | | | MA | 54079 | A | 08 September 2021 |
| | | | | AU | 2019374103 | A1 | 20 May 2021 |
| | | | | KR | 20210102888 | A | 20 August 2021 |
| CN | 110462038 | A | 15 November 2019 | BR | 112019016204 | A2 | 07 July 2020 |
| | | | | KR | 20190133160 | A | 02 December 2019 |
| | | | | EP | 3581651 | A1 | 18 December 2019 |
| | | | | PH | 12019501824 | A1 | 14 September 2020 |
| | | | | MX | 2019009358 | A | 02 December 2019 |
| | | | | ZA | 201905905 | B | 25 May 2022 |
| | | | | IL | 268588 | A | 26 September 2019 |
| | | | | SG | 10201912368 X | A | 27 February 2020 |
| | | | | SG | 11201907321 T | A | 27 September 2019 |
| | | | | WO | 2018147245 | A1 | 16 August 2018 |
| | | | | AU | 2018218753 | A1 | 26 September 2019 |
| | | | | TW | 201837174 | A | 16 October 2018 |
| | | | | CA | 3052938 | A1 | 16 August 2018 |
| | | | | RU | 2019128134 | A | 09 March 2021 |
| | | | | JP | WO2018147245 | A1 | 21 November 2019 |
| | | | | US | 2019367612 | A1 | 05 December 2019 |
| | | | | CO | 2019009680 | A2 | 07 February 2020 |
| CN | 111788231 | A | 16 October 2020 | TW | 201936641 | A | 16 September 2019 |
| | | | | RU | 2020129004 | A | 09 March 2022 |
| | | | | KR | 20200119833 | A | 20 October 2020 |
| | | | | EP | 3749692 | A1 | 16 December 2020 |
| | | | | PE | 20201341 | A1 | 25 November 2020 |
| | | | | IL | 276537 | A | 30 September 2020 |
| | | | | CO | 2020008940 | A2 | 31 August 2020 |
| | | | | MA | 51734 | A | 19 May 2021 |
| | | | | JP | 2021513334 | A | 27 May 2021 |
| | | | | CR | 20200341 | A | 02 November 2020 |
| | | | | AU | 2019219061 | A1 | 06 August 2020 |
| | | | | WO | 2019154890 | A1 | 15 August 2019 |
| | | | | US | 2021054094 | A1 | 25 February 2021 |
| | | | | CL | 2020001854 | A1 | 23 October 2020 |
| | | | | SG | 11202007578 S | A | 29 September 2020 |
| | | | | BR | 112020015297 | A2 | 08 December 2020 |
| | | | | CA | 3088730 | A1 | 15 August 2019 |
| | | | | AR | 117392 | A1 | 04 August 2021 |
| | | | | PH | 12020551211 | A1 | 17 May 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107428829 A